(19) Europäisches Patentamt
European Patent Office
Office européen des brevets

(11) EP 2 114 456 B1

## (12) EUROPEAN PATENT SPECIFICATION

(45) Date of publication and mention
of the grant of the patent:
**04.03.2015 Bulletin 2015/10**

(21) Application number: **08700170.7**

(22) Date of filing: **11.01.2008**

(51) Int Cl.:
*A61K 47/18* $^{(2006.01)}$ *A61K 47/14* $^{(2006.01)}$
*A61K 47/22* $^{(2006.01)}$ *A61K 38/44* $^{(2006.01)}$
*A61K 38/27* $^{(2006.01)}$ *A61K 39/29* $^{(2006.01)}$
*A61K 9/08* $^{(2006.01)}$

(86) International application number:
**PCT/GB2008/000082**

(87) International publication number:
**WO 2008/084237 (17.07.2008 Gazette 2008/29)**

(54) **STABILIZATION OF AQUEOUS COMPOSITIONS OF PROTEINS WITH DISPLACEMENT BUFFERS**

STABILISIERUNG WÄSSRIGER PROTEINZUSAMMENSETZUNGEN MIT VERDRÄNGUNGSPUFFERN

STABILISATION DE FORMULATIONS AQUEUSES DE PROTEINES AVEC DES TAMPONS DE DEPLACEMENT

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **11.01.2007 GB 0700523**
**31.05.2007 US 941125 P**

(43) Date of publication of application:
**11.11.2009 Bulletin 2009/46**

(73) Proprietor: **Arecor Limited**
**Cambridge CB4 0FE (GB)**

(72) Inventor: **JEZEK, Jan**
**Bedfordshire MK44 1LQ (GB)**

(74) Representative: **Teuten, Andrew John et al**
**Sagittarius IP**
**Three Globeside**
**Fieldhouse Lane**
**Marlow, Buckinghamshire SL7 1HZ (GB)**

(56) References cited:
**EP-A- 0 884 053      EP-A- 0 938 902**
**EP-A- 1 506 786      WO-A-2008/066322**
**US-A- 4 476 118      US-A1- 2005 272 657**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

Field of the Invention

This invention relates to the stability of proteins, particularly the stability of proteins in aqueous systems, for example in aqueous solution or in aqueous gel form.

Background to the Invention

Many proteins, e.g., enzymes, antibodies or therapeutic proteins are unstable and are susceptible to structural degradation and consequent loss of activity while stored, particularly in aqueous solutions. The processes involved in protein degradation can be divided into physical (i.e. processes affecting non-covalent interactions, such as loss of quaternary, tertiary or secondary structure, aggregation, surface adsorption) and chemical (i.e. processes involving a covalent change such as de-amidation, oxidation, disulphide scrambling etc.). The rates of the degradation processes are proportional to temperature. Proteins are consequently generally more stable at lower temperatures.

In general, proteins are more stable in the absence of water. Most commercial proteins are therefore formulated as lyophilised powders. A typical lyophilised commercial protein formulation always comprises a buffer, such as phosphate buffer, and one or more additives. The additives may include one or more of the following:

- Bulking agents: typically sugars or sugar alcohols such as sorbitol or mannitol
- Stabilisers: typically water replacement sugars such as trehalose or sucrose that can protect the protein structure during freeze-drying.
- Tonicit modifiers: typically inorganic salts and amino acids (commonly glycine or arginine). These excipients are used to adjust ionic strength. Ionic strength is often an important parameter of the protein formulation both during the freeze-drying process and in the specific application of the protein following reconstitution.
- Surfactants: may be effective to prevent adsorption of proteins onto solid surfaces, agitation-induced aggregation and damage during freeze-drying.

Some proteins are known to be formulated in solutions. Historically, this reduces production cost considerably at the expense of low stability. Aqueous solutions of proteins are often formulated in early stage development of a protein product during which the stability demands are not as strict as those for the final product. Typically, aqueous protein solutions have to be stored strictly at 4°C. In most cases, structural degradation and loss of activity occur even at this temperature over a period of storage. The stability of aqueous formulations can be improved by freezing, but in some cases the freeze-thaw cycle can contribute to the protein damage.

A typical aqueous protein solution is formulated in a conventional buffer, most commonly in phosphate buffer pH 6.8 - 7.3, although other buffers such as HEPES, TRIS, carbonate or citrate are also used. The formulations may also comprise one or more of the following additives:

- Tonicity modifiers: typically inorganic salts and amino acids (commonly glycine or arginine). These excipients are used to adjust ionic strength, an important parameter of the protein formulation.
- Surfactants: may be effective to prevent adsorption of proteins onto solid surfaces or agitation-induced aggregation.
- Stabilizers: typically water replacement sugars such as trehalose or sucrose.

These are known to affect the melting point of proteins and may consequently improve the protein stability.

As shown above, the nature of additives in commercial protein formulations can vary. However, the common feature of the commercial formulations of proteins both in dry and in aqueous format is the presence of a buffer. A buffer is required to maintain the pH of the formulation close to a given value. Many commercial proteins are formulated in phosphate buffer at pH close to 7. In some cases, other buffers and other pH can be used. Formulating at pH away from 7 is typically driven by the need to increase protein solubility, which can be achieved at pH away from the isoelectric point of the protein.

The choice of buffer for formulating proteins follows the well-defined rules of acid-base equilibria and Brønsted-Lowry acid-base theory. Acid-base equilibria relate to the exchange of protons ($H^+$; also referred to as hydrogen cations) between two chemical species. Whilst the species that is donating the proton is referred to as the acid, the species that is accepting the proton is referred to as the base. So, in the following reversible process,

$$HA + B^- \rightleftarrows HB + A^-$$

HA acts as acid and $B^-$ acts as base. In the opposite direction HB acts as acid and A- acts as base. The ability of a compound to donate or accept proton is expressed by the dissociation constant $K_a$ which describes the equilibrium between the protonated and de-protonated form of a compound in aqueous solutions as follows:

$$HX + H_2O \rightleftarrows H_3O^+ + X$$

$$K_{eq}=[H^3O^+][X^-]/[HX][H_2O]$$

Since the $[H_2O]$ = Constant = 55.5 M then:

$$K_a = K_{eq}[H_2O]=[H_3O^+][X^-]/[HX]$$

$$pK_a = -\log K_a$$

The $pK_a$ of any species is a function of temperature. Whilst in many cases, such as phosphate, citrate or acetate, the temperature dependence is small, some buffers (such as TRIS/HCl) exhibit change of $pK_a$ by as much as 0.03 unit per each °C.

The degree of protonation of a chemical species with a given $pK_a$ value depends on the pH of the solution. If pH = $pK_a$ of the species in question then 50% of the species exists in the protonated form and the remaining 50% in de-protonated form. If pH is one unit lower than $pK_a$ then 90% of the species exists in the protonated form and 10% in the de-protonated form. Similarly, if pH is one unit higher than $pK_a$ then 10% of the species exists in the protonated form and 90% in the de-protonated form. Although the percentage of the protonated and de-protonated forms of a compound remains constant so long as the pH and the temperature are constant, this is a result of a dynamic equilibrium between the compound and surrounding molecules. In other words, there is a continuous dynamic exchange of protons between the acid-base species in a system while the overall protonation status of each species in the solution is maintained constant.

By donating or accepting protons in the pH range around its $pK_a$ the species acts as a buffer. The presence of a buffer thus results in small changes of pH if either an acid or a base is added to the solution. The species exerts maximum buffering capacity at pH = $pK_a$, and its ability to maintain pH declines as the pH moves away from the $pK_a$.

The choice of the appropriate buffer generally depends on the pH required. The generally accepted rule is that the $pK_a$ of the buffer must be no more than one unit away from the required pH to act as an efficient buffer. Preferably, however, the $pK_a$ is within 0.5 units away from the required pH in order to maximise the buffering capacity of the species. Most preferably the $pK_a$ of the buffer is equal to the required pH of the solution. In this case, the proportion of the protonated form and the deprotonated form of the buffer are 50% respectively and its buffering capacity is utilised to the full extent. Such solution is then most efficiently protected against changes of pH both in the acid and in the alkaline direction.

EP1314437 discloses an aqueous composition comprising an antibody and histidine, at pH 7.1. This composition is said to be stable with respect to aggregation. Subsequent description suggests that, for use, a buffer should be added.

PCT/GB2006/002470 describes an aqueous system comprising a protein and one or more stabilising agents. The stabilising agents have ionisable groups capable of exchanging protons with the protein and with the ionised product of water dissociation. The ionisable groups include first groups that are positively charged when protonated and uncharged when deprotonated, and second groups that are uncharged when protonated and negatively charged when deprotonated. The pH of the composition is within a range of protein stability that is at least 50% of the maximum stability of the protein with respect to the pH; alternatively, the pH of the composition is no more than 0.5 units more or less than the pH at which the composition has maximum stability with respect to pH. The disclosure is based on the observation that, while there is invariably a range of pH values for which a composition is relatively stable, the presence of certain excipients is desirable. It is stated that a buffer may be added.

EP884053 describes a monomeric insulin formulation stabilised against aggregation in which the buffering agent is either TRIS or arginine.

US4476118 describes a solution of insulin with improved stability containing zinc ions.

However, a need exists to improve the stability of aqueous protein solutions.

Summary of the Invention

The present invention is based on the discovery that buffers having a pKa at or near the pH of the solution are undesirable, when considering the protein's stability with respect to pH. Rather, the key to the present invention is choice of the appropriate pH while minimizing the protein's ability to exchange ions. Various aspects of the invention are defined in the claims.

The invention relates to a sealed container comprising an aqueous composition comprising a protein and two displacement buffers, wherein one displacement buffer has a pKa that is at least 1 unit greater than the pH of the composition and one displacement buffer has a pKa that is at least 1 unit less than the pH of the composition, said pH of the composition being set to a value at which the composition has maximum measurable stability with respect to pH, wherein one of the displacement buffers is TRIS, with the proviso that said composition contains not more than 2 mM of a conventional buffer having a pKa that is within one pH unit of the pH of the composition, and wherein each displacement buffer is present at a concentration of 2 to 200 mM.

By keeping a protein at a suitable pH, at or near a value at which the measurable stability is maximal, in the absence of a conventional buffer, the storage stability of the protein can be increased substantially. Storage stability can generally be enhanced further, possibly substantially, by use of additives with $pK_a$ values having 1 to 5 units away from the pH of the composition at the intended temperature range of storage of the composition. The presence of these additives also improves the pH stability of the formulation and is generally preferred.

In accordance with the present invention the protein composition does not comprise a conventional buffer in a meaningful amount. In other words, the protein composition contains less than a meaningful amount of the conventional buffer. Conventional buffers are typically applied in protein compositions at concentrations 2-200 mM, more typically at 5 - 50 mM and most typically at about 20 mM concentration. The term "conventional buffer" is therefore defined herein as any chemical species with $pK_a$ less than one unit but preferably less than 0.5 units away from pH of the composition as measured at the intended temperature range of storage of the composition which possesses a buffering capacity for the protein. The term "less than a meaningful amount" means that the conventional buffer is present in the composition at concentration less than 2 mM.

The composition contains two additives capable of engaging in acid-base equilibria with $pK_a$ values at least 1 unit below the pH of the composition and with $pK_a$ values at least 1 unit above the pH of the composition. As used herein, one or more units above or below the pH of the composition are also referred to herein as 1 or more units "away" from the pH of the composition. Such additives can protect the composition from significant shifts of pH either toward acidic values (if $pK_a$ is lower than pH of the composition) and toward alkaline values (if $pK_a$ is higher than pH of the composition).

Such additives are suitably present in an amount such that the molarity of each additive is 2 mM to 200 mM, most preferably 5 mM to 100 mM. In one embodiment, two additives are present at a concentration of 2mM to about 200mM, more preferably at a concentration from about 5mM to about 100mM.

Apart from the additives capable of exchanging protons with the said protein, the composition may contain other excipients in order to meet the requirements for the intended use of the formulation. Examples of such excipients include inorganic salts to adjust the ionic strength, surfactants to minimise surface adsorption of the proteins, preservatives to maintain the composition in sterile condition, chelating agent to complex metals or a protease inhibitor to ensure that the protein is not slowly digested by protease activity present in the sample. Another additive that may be used is a polyalcohol, e.g. at a concentration of at least 0.5%, and typically up to 5% (w/w). Examples of such compounds are saccharides such as sucrose or trehalose or sugar alcohols such as inositol, lactitol, mannitol or xylitol.

The protein that is stabilized in accordance with the invention, may be in a microbiologically sterile form, and is conveniently contained or stored in a sealed, sterile container such as a vial, syringe or capsule and stored at a desired temperature.

Brief Description of the Drawings

Fig. 1 is a graph showing the relative concentrations of the buffering species of a conventional buffer (A) and displaced buffers (B 1 and B2) in a hypothetical system to buffer a composition at pH 7. $pK_a(A) = 7$, $pK_a(B1) = 5$, $pK_a(B2) = 9$. The dotted lines show the relative concentration of the de-protonated forms of the buffers (i.e. the form capable of preventing pH changes into acidic values); the full lines show the relative concentration of the protonated forms of the buffers (i.e. the form capable of preventing pH changes into alkaline values).

Fig. 2 is a titration curve of the composition comprising either 20 mM conventional buffer (pKa 7) or two displacement buffers, (pKa 5 and pKa 9), both at 20 mM concentration. The acid titration is expressed as negative addition of hydroxide anions.

Fig. 3 is a titration curve of the composition comprising either 20 mM conventional buffer (pKa 7) or two displacement buffers, (pKa 5 and pKa 9), both at 100 mM concentration.

Detailed Description of the Invention

It will be appreciated that this invention relates to the stability of proteins, particularly the stability of proteins in an aqueous environment, e.g. in aqueous solution or in aqueous gel form and concerns the storage stability of proteins, e.g. stability with time, including stability at ambient temperatures (about 20°C) and above.

The term "protein" is used herein to encompass molecules or molecular complexes consisting of a single polypeptide, molecules or molecular complexes comprising two or more polypeptides and molecules or molecular complexes comprising one or more polypeptides together with one or more non-polypeptide moieties such as prosthetic groups, cofactors etc. The term "polypeptide" is intended to encompass polypeptides comprising covalently linked non-amino acid moieties such as glycosylated polypeptides, lipoproteins etc. In particular, the invention relates to molecules having one or more biological activities of interest, which activity or activities are critically dependent on retention of a particular or native three-dimensional structure in at least a critical portion of the molecule or molecular complex.

The present invention enables improvements to be made in the storage stability of proteins by selecting an appropriate pH of the composition without the use of a conventional buffer. The term "conventional buffer" is used herein to encompass any compound possessing a buffering capacity when present in the composition with $pK_a$ less than one unit away from the pH, but preferably less then 0.5 unit, most preferably with $pK_a$ = pH. Both the $pK_a$ and the pH values used in this definition are those measured at the temperature range of the intended storage of the protein composition.

Conventional buffers are typically present in protein compositions at concentrations 2-200 mM, more typically at 5 - 50 mM and most typically at about 20 mM concentration. Such concentrations of conventional buffers can ensure reasonable stability of pH and can therefore be referred to as meaningful concentrations with respect to their buffering action. Consequently, apart from the above specification in terms of its $pK_a$, the term "conventional buffer" additionally comprises a "meaningful concentration" aspect characterized in that the said conventional buffer is present at a concentration that is meaningful with respect to a reasonable buffering action. In other words, a meaningful concentration of a conventional buffer is that concentration wherein the conventional buffer provides the predominant buffering mechanism of the system.

The present invention arose from an analysis of the effects of chemical species capable of proton exchange on stability of proteins and the subsequent development of a model that enables selection of conditions that ensure good long-term stability of proteins. The analysis revealed that the presence of acid-base species that are close to 50% protonation state is detrimental to the protein stability as determined by either functional assays or structural assays. By definition, this means that the presence of conventional buffers, especially at high concentrations, can be detrimental to the protein stability. It appears that, prior to the present invention, the adverse effect of conventional buffers on the storage stability of proteins has not been appreciated.

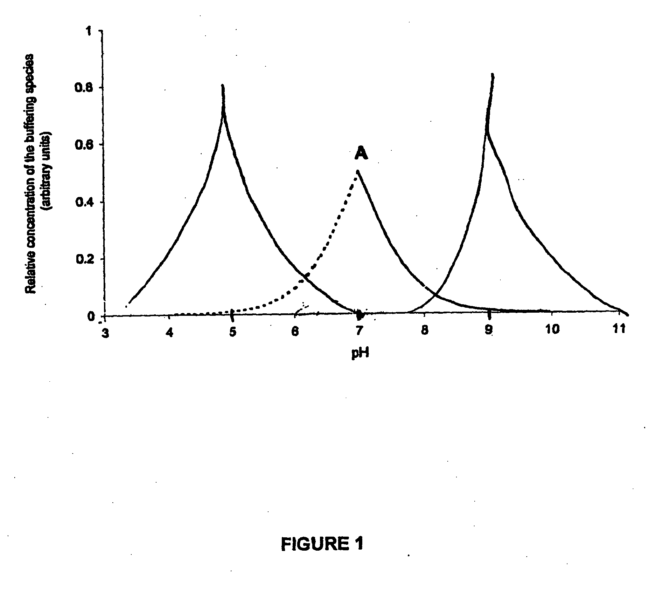

Some limited buffering capacity can be derived from the protein itself, especially in the pH range of 4.0 to 6.5 due to the side chains of aspartic acid, glutamic acid and histidine. In some cases, especially at higher protein concentrations (> 20 mg mL$^{-1}$) this might be sufficient to maintain the required pH, especially in sterile composition in which spontaneous changes of pH are unlikely. In accordance with the invention, one or more excipients or additives can be used to maintain the required pH or minimise pH changes. This can be referred to as "displaced buffering" and is based on addition of excipients to the protein composition with $pK_a$ values outside the conventional buffering range, preferably excipients with $pK_a$ about 1 to 4 units above or below the pH of the composition. Although the "displaced buffering" cannot ensure a strong buffering capacity at the required pH comparable with the conventional buffer, it can still prevent significant fluctuations of pH away from the required value. The difference between conventional buffering and displaced buffering is shown in Fig. 1. The graph shows the relative concentrations of the buffering species of a conventional buffer (A) and displaced buffers (B1 and B2) in a hypothetical system buffered at pH 7. The dotted lines show the relative concentration of the de-protonated forms of the buffers (i.e. the form capable of preventing pH changes into acidic values); the full lines show the relative concentration of the protonated forms of the buffers (i.e. the form capable of preventing pH changes into alkaline values). The concentration of the buffering species on both the acidic and the alkaline side reflects the buffering capacity of the buffer. The conventional buffer (in this case a compound with $pK_a$ = 7) is most effective to maintain the required pH 7. The two displacements buffers (in this case compounds with $pK_a$s two units above and two units below the required pH) exert minimal buffering capacity at pH 7, but their buffering capacity increases as pH moves away from 7. So, whilst these species are rather inefficient in preventing small fluctuations around the required pH they can prevent larger fluctuations away from the required pH. The ability of the displacement buffers to maintain pH away from their respective $pK_a$ values increases with their concentration as shown in Fig. 1.

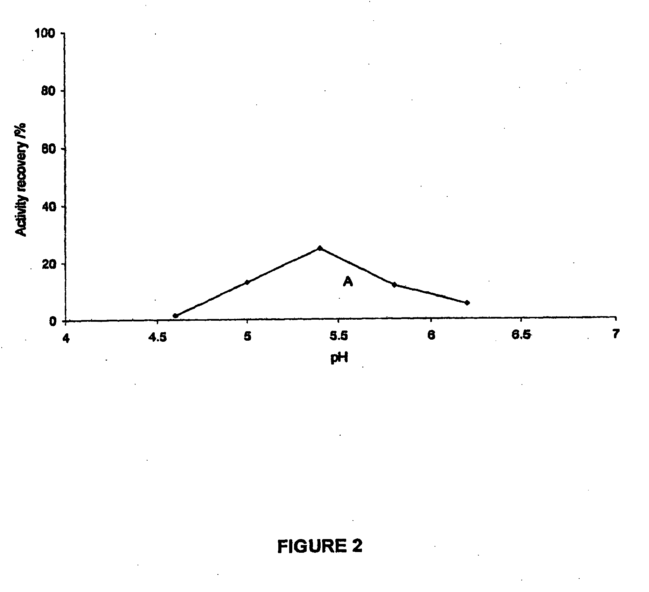

The titration curve of the composition titrated with either a base (OH$^-$) or an acid (H$_3$O$^+$) is shown in Fig. 2. In this model example the target pH is 7, and the titration is shown of a composition comprising 20 mM of a conventional buffer ($pK_a$ 7) and a combination of two displaced buffers ($pK_a$ 5 and $pK_a$ 9), each at 20 mM concentration. The titration curves are theoretical ones, based on the $pK_a$ values and concentrations of the species present and on the assumption that no other components of the composition contribute to the buffering of the composition.

Due to a limited buffering capacity of the displaced buffers at the target pH the slope of the titration curve at the target pH (i.e. 7 in this model example) in the composition containing conventional buffer is considerably less steep compared to that containing the combination of displaced buffers. Consequently, addition of the same amount of NaOH will cause different pH change in the presence of conventional buffer compared with that in the presence of displacement buffers of the same concentration. So, in the model example shown in Fig. 2 (where the pKa of conventional buffer is precisely the same as the target pH 7) the addition of 5 mM NaOH (i.e. addition of NaOH to the composition, which results in 5 mM concentration increase of Na$^+$ cations in the composition) will increase pH from 7.0 to 7.48 in the presence of conventional buffer (20 mM) and to 8.55 in the presence of displacement buffers (both at 20 mM).

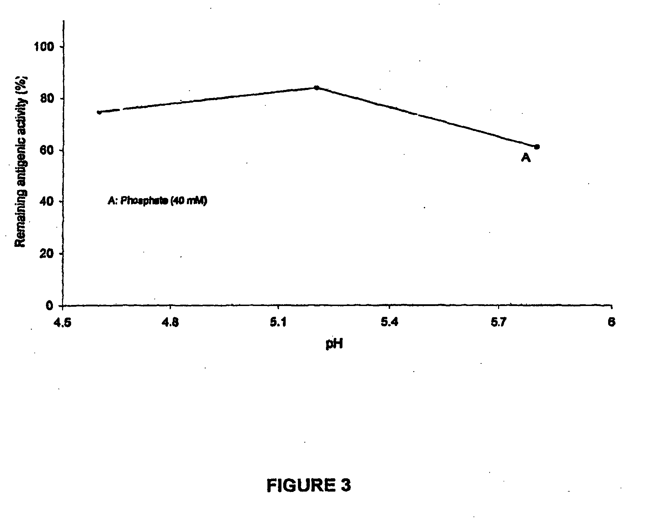

However, one skilled in the art will appreciate that the buffering efficiency of the displaced buffer at the target pH can be increased by increasing the concentration of the displaced buffers. This is illustrated in Fig. 3 for a 20 mM conventional buffer and a combination of displaced buffers (pKa 5 and pKa 9), each at 100 mM concentration (compare with Fig. 2 where the same situation is illustrated for 20 mM displaced buffers). Similarly, the buffering efficiency of the conventional buffer is proportional to the buffer concentration.

The buffering efficiency depends on the slope of the titration curve at the target pH. The lower the slope the better buffering efficiency is achieved. The slope of the titration curves in the above model examples at the target pH (i.e. pH 7) is approximately as follows:

$$\frac{d\,pH}{d\,[OH^-]} = 0.017 \text{ mM}^{-1} \text{ in the case of conventional buffer (100 mM)}$$

$$\frac{d\,pH}{d\,[OH^-]} = 0.087 \text{ mM}^{-1} \text{ in the case of conventional buffer (20 mM)}$$

$$\frac{d\,pH}{d\,[OH^-]} = 0.349 \text{ mM}^{-1} \text{ in the case of conventional buffer (5 mM)}$$

$$\frac{d\,pH}{d\,[OH^-]} = 0.222 \text{ mM}^{-1} \text{ in the case of displacement buffers (each at 100 mM).}$$

$$\frac{d\,pH}{d\,[OH^-]} = 1.111 \text{ mM}^{-1} \text{ in the case of displacement buffers (each at 20 mM)}$$

$$\frac{d\,pH}{d\,[OH^-]} = 4.369 \text{ mM}^{-1} \text{ in the case of displacement buffers (each at 5 mM)}$$

So, although in general the buffering efficiency of displaced buffers is considerably lower in the case of displacement buffers compared with that of conventional buffers, it can be compensated by increasing the concentration of the displaced buffers. For example, the buffering efficiency of displaced buffers (pK$_a$s 5 and 9) at 100 mM concentrations result in better buffering efficiency compared with that achieved by conventional buffer (pKa 7) at 5 mM concentration.

One embodiment of the present invention includes a protein composition which is "buffered essentially" by the displacement buffer. This means that the slope of the titration curve $\left(\frac{d\,pH}{d[OH^-]}\right)$ at the storage pH of the composition brought about by all ionisable groups of the composition having pK$_a$ at least one unit away from the pH of the composition is substantially lower than that brought about by all ionisable groups of the composition having pKa less than one unit away from the pH of the composition. This means that the displacement buffer contributes substantially more to the pH-buffering of the composition than the conventional buffer. The slope of the titration curve $\left(\frac{d\,pH}{d[OH^-]}\right)$ at the storage pH of the composition brought about by all ionisable groups of the composition having pK$_a$ at least one unit away from the pH of the composition must be at least twice as low, preferably 5 times lower, most preferably 10 times lower than that brought about by all ionisable groups of the composition having pKa less than one unit away from the pH of the composition.

One skilled in the art will be able to determine the slope of the titration curves either theoretically based on the concentrations of all ionisable groups in the composition and their $pK_a$s. Alternatively, the slope of the titration curves can be confirmed experimentally by measuring the titration curve in two compositions in which the compounds with ionisable groups having pKa less than one unit away and more than one unit away from the pH of the composition are separated.

In one embodiment the protein composition of the sealed container of the invention comprises two displacement buffers comprising at least one displacement buffer having a pKa that is at least 1.5 units greater than the pH of the composition at the desired temperature and at least one displacement buffer having a pKa that is at least 1.5 units less than the pH of the composition at the desired temperature. In one embodiment the protein composition of the sealed container of the invention comprises two displacement buffers comprising at least one displacement buffer having a pKa that is at least 2 units greater than the pH of the composition at the desired temperature and at least one displacement buffer having a pKa that is at least 2 units less than the pH of the composition at the desired temperature.

In one embodiment the protein composition of the sealed container of the invention comprises two displacement buffers wherein each displacement buffer has a pKa from about 1 unit to about 5 units from the pH at which the protein has stability at the desired temperature. In one embodiment the protein composition of the sealed container of the invention comprises two displacement buffers wherein each displacement buffer has a pKa from about 1 unit to about 4 units from the pH at which the protein has stability at the desired temperature. Apart from the contribution to pH buffering, the presence of displacement buffers was shown in many cases to have a beneficial effect on the protein stability. For example, in one embodiment, protein activity of a protein in a composition in accordance with the invention retains at least 40% of its activity for at least one week, and preferably at least four weeks at a desired temperature (e.g. ambient temperature or higher). In another embodiment, protein activity of a protein in a composition in accordance with the sealed container of the invention retains at least 50% of its activity for at least one week at the desired temperature, and preferably at least four weeks at a desired temperature (e.g. ambient temperature or higher). In another embodiment, at least 40% and preferably at least 50% protein structural activity of a protein present in a composition according to the sealed container of the invention is retained for at least one week and more preferably for at least 4 weeks at the desired temperature.

The compounds that can be used as displacement buffers can be both organic and inorganic. They can be of both monomeric and polymeric nature.

Some examples of compounds that can be usefully incorporated in the protein composition as additives and that may possibly also function as displacement buffers are known and include, but are not limited to: Histidine, Maleate, Sulphite, Cyclamate, Hydrogen sulphate, Serine, Arginine, Lysine, Asparagine, Methionine, Threonine, Tyrosine, Isoleucine, Valine, Leucine, Alanine, Glycine, Tryptophan, Gentisate, Salicylate, Glyoxylate, Aspartame, Glucuronate, Aspartate, Glutamate, Tartrate, Gluconate, Lactate, Glycolic acid, Adenine, Succinate, Ascorbate, Benzoate, Phenylacetate, Gallate, Cytosine, p-Aminobenzoic acid, Sorbate, Acetate, Propionate, Alginate, Urate, 2-(N-Morpholino)ethanesulphonic acid, Bicarbonate, Bis(2-hydroxyethyl) iminotris(hydroxymethyl)methane, N-(2-Acetamido)-2, iminodiacetic acid, 2-[(2-amino-2-oxoethyl)amino]ethanesulphonic acid, Piperazine, N,N'-bis(2-ethanesulphonic acid), Phosphate, N,N-Bis(2-hydroxyethyl)-2, aminoethanesulphonic acid, 3-[N,N-Bis(2-hydroxyethyl)amino]-2, hydroxypropanesulphonic acid, Triethanolamine, Piperazine-N,N'-bis(2, hydroxypropanesulphonic acid), Tris(hydroxymethyl)aminomethane, N, Tris(hydroxymethyl)glycine, N-Tris(hydroxymethyl)methyl-3-aminopropanesulphonic acid, Ammonium ion, Borate, 2-(N-Cyclohexylamino)ethanesulphonic acid, 2-Amino-2-methyl-1-propanol, Palmitate, Creatine, Creatinine, and salts thereof.

The particular choice of the compound will depend on pH of the composition. So, for example, purine (pKa = 9.0) is a suitable additive in a composition at pH 7.0 (where pKa - pH = 2.0), but not in a composition at pH 8.8 (where pKa - pH = 0.2 and purine therefore becomes the conventional buffer). It will of course be understood by one of ordinary skill in the art that aspects specific to particular proteins have to be taken into account. For instance, it is important to ensure that the additives selected do not inhibit the protein activity. Many of the suggested additives are GRAS (Generally Regarded As Safe) or approved ingredients in pharmaceutical products. These additives are particularly suitable for stabilisation of proteins in pharmaceutical compositions. Other applications where safety is not of major concern, such as proteins in diagnostic kits, may rely on compounds outside the GRAS category.

In one embodiment, the invention includes a sealed container containing an aqueous composition comprising a protein and two displacement buffers having a pKa at least 1 unit more or 1 unit less than the pH of the composition wherein such composition is buffered essentially by the one or more displacement buffers. A composition is "buffered essentially" by one or more displacement buffers when such displacement buffers are present at a concentration that provides the predominant buffering mechanism of the system.

In addition to allowing the presence of certain materials that affect the pH, the composition may include components other than those of the displacement buffers or additives. For example, compositions may include inorganic salts e.g. to adjust the ionic strength of the composition, a sugar or sugar alcohol, a preservative, a protease inhibitor, a chelating agent, an ionic detergent or non-ionic detergent. The difference between the pH of the composition and the pKa of the "displacement buffer" used in this invention is at least 1 unit, preferably at least 1.5, e.g. at least 2 and up to 5 or more.

It is to be understood that conventional buffer, i.e. that buffer which does not meet the definition of a displacement buffer, may be used so long as the displacement buffer provides the predominant buffering mechanism of the system, i.e. at least 50%, but preferably at least 80% buffering capacity of the system.

Without wishing to be bound by theory, it is believed that the beneficial effect of the present invention on the protein stability is due to the fact that at or around the 50% protonation state the acid-base species are most likely to exchange protons with surrounding acid-base species, such as some amino acids at the protein surface. Such exchanges can be detrimental to the protein for the following reasons:

- Each proton exchange results in either bond formation or bond cleavage. Such processes are accompanied by energy exchanges (e.g. translational energy of the species involved) between the protein and surrounding species and by changes in charge characteristics of the part of the protein where proton is exchanged. Therefore, the continuous proton exchanges occurring when protein is in equilibrium with its aqueous environment are very likely to contribute to the fluctuations of the protein structure and consequent physical instability of the protein.

- Various chemical processes affecting protein stability, such as de-amidation, involve proton exchange. Minimising the rate of these processes can therefore lead to stabilisation of the protein.

The invention is applicable to proteins dissolved freely in aqueous solutions or aqueous gel forms or to proteins present in an aqueous system as a dispersion or suspension, as well as proteins attached to solid substrates such as vaccine adjuvant or cellular membrane by means of hydrophobic, ionic or ligand exchange interactions.

The invention is applicable to stabilization of a protein throughout its product life including isolation or expression, purification, transport and storage.

In terms of secondary structure, the invention is applicable to proteins containing any proportion of alpha helix, beta sheet and random coil.

In terms of tertiary structure, the invention is applicable both to globular proteins and to fibrillar proteins. The invention is applicable to proteins whose tertiary structure is maintained solely by means of non-covalent interactions as well as proteins whose tertiary structure is maintained by combination of non-covalent interactions and one or more disulphide bridges.

In terms of quaternary structure, the invention is applicable to monomeric proteins as well as proteins consisting of two, three, four or more subunits. The invention is also applicable to protein conjugates.

In terms of non-protein structural components, the invention is applicable to proteins that do not contain any non-peptide components as well as glycoproteins, lipoproteins, nucleoproteins, metalloproteins and other protein containing complexes where protein represents at least 10% of the total mass. It is applicable to proteins that do not require a cofactor for their function as well as to proteins that require a coenzyme, prosthetic group or an activator for their function.

The protein may be native or recombinant, glycosylated or non-glycosylated, autolytic or non-autolytic. The invention is particularly applicable to the following groups of proteins.

**Protein or peptide hormones and growth factors**

The function of protein or peptide hormones and growth factors depends on their ability to bind to a specific receptor. Such binding event is linked closely to the protein conformation. The retention of three-dimensional structure of the protein, or at least the 3-D structure of key domains, is therefore crucial for their function. The retention of structural and functional characteristics is also of paramount importance for the regulatory approval of the protein therapeutics. Examples of therapeutic protein or peptide hormones include:

Insulin (treatment of diabetes)
Glucagon (treatment of diabetes)
Human growth hormone
Gonadotropin
Human thyroid stimulation hormone (treatment of thyroid cancer)
Granulocyte colony stimulation factor (used as part of chemotherapy)

**Therapeutic enzymes**

The function of therapeutic enzymes depends directly on their molecular structure and conformation. Irreversible conformational changes and irreversible aggregation lead to inactivation of the therapeutic enzymes. The retention of the structural characteristics of the protein is also an essential pre-requisite of the regulatory approval. Examples of therapeutic protein or peptide hormones include:

Streptokinase (thrombolytic agent in treatment of ischemic stroke)
Asparaginase
Urate oxidase
Papain (tissue debridement).

**Vaccines**

The immunogenic activity of protein vaccines depends (to a large extent) on the structural integrity of the key protein antigens, especially in relation to conformational epitopes (where antibodies are required to bind disparate regions of the polypeptide chain brought together by native folding). Irreversible conformational changes and irreversible aggregation lead to inactivation of vaccines. The same considerations apply to proteins adsorbed onto particles, such as alumina particles, or other (non-particulate) surfaces when substantial regions of each protein molecule are still in full interaction with solvent water. This is of particular importance in vaccine distribution in the third world where the maintenance of the cold chain is very difficult or impossible, partly through practical or logistic limitations and partly through cost. The present invention can be applied to recombinant protein vaccines as well as attenuated viruses or whole cell vaccines, providing the key antigens consist of polypeptide chains. Examples of such vaccines include:

Hepatitis B vaccine
Malaria vaccine
Human papilloma vaccine
Meningitis A vaccine
Meningitis C vaccine
Pertussis vaccine
Polio vaccines

**Therapeutic antibodies**

The function of therapeutic antibodies is based on their specific interactions with target antigens. So, in order to maintain their function, the retention of the three-dimensional structure is essential for the duration of their shelf life. Although generally very stable at ambient temperature, due to the inherent rigid, stable structure of the immunoglobulin fold or domain, antibodies can benefit from the present invention, by further increasing their stability in storage. Examples of therapeutic antibodies that can be used, for example, in cancer therapy include:

anti-Epidermal Growth Factor Receptor (EGFR) monoclonal antibody
anti-HER2 monoclonal antibody (breast cancer therapy)
anti-CD52 monoclonal antibody (chronic lymphocytic leukaemia therapy)
anti CD20 monoclonal antibody (aggressive lymphoma therapy)

**Interferons**

Interferons are rather unstable polypeptides of therapeutic importance that are used, for example, in multiple sclerosis therapy. Application of the present invention can increase the shelf life and cost effectiveness of interferons, including interferon alpha, interferon beta and interferon gamma.

**Other therapeutic proteins**

Following are examples of other therapeutic proteins that can benefit from the application of the present invention, in terms of cost-effectiveness and improved shelf life, particularly in aqueous solution:

Erythropoietin (stimulating erythrocyte production)
Darbepoietin alpha (stimulating erythrocyte production)
Blood coagulation factors, mainly Factor VIII and Factor IX (treatment and control of haemophilia)
Immunosuppressive agents (treatment of various conditions such as asthma, allergic rhinitis or multiple sclerosis)
Human albumin
Protein C (antithrombic agent)

## Diagnostic and industrial proteins

The retention of the structural characteristics is crucial for the function of diagnostic proteins, particularly enzymes and antibodies. In particular, in-kit reference standards of the analytes, through which the assay is calibrated and subjected to QC, must be rigorously stabilized, as any drift in their integrity will cause a resultant drift in accuracy of the whole kit. Impaired activity can lead to false results or poor performance (e.g. slow running of the procedure). Stability of the functional activity of diagnostic proteins throughout their product life is therefore of paramount importance. Manufacturers of diagnostic products are keen to find approaches and formulations that would eliminate costly lyophilisation, which causes substantial processing bottle-necks. Examples of diagnostic proteins include:

Monoclonal antibodies
Polyclonal antibodies
Antibody-enzyme conjugates
Oxidases such as glucose oxidase, galactose oxidase, cholesterol oxidase
Peroxidases
Alkaline phosphatase
Dehydrogenases such as glutamate dehydrogenase, glucose dehydrogenase
Isomerases such as invertase
Hydrolases such as trypsin, or chymotrypsin

Integral assay reference standards supplied in kit form, such as hormones, growth factors, microbial proteins, metabolic proteins, soluble forms of structural proteins etc.
Examples of industrial proteins include:

Amylase
Protease
Lipase

## International reference standards of therapeutic proteins, vaccines and diagnostics

Reference standards of therapeutic or diagnostic proteins are of great importance for standardization of therapeutic and diagnostic procedures. The stability of reference standards is of fundamental importance. A wide range of protein-based reference standards are therefore an ideal target for the present invention.
In one embodiment of this disclosure there is provided an aqueous protein composition wherein the protein has a pH of 6 at the desired temperature, and the protein composition comprises at least one displacement buffer having a pKa that is 7 or greater and preferably such displacement buffer is selected from TRIS, purine and cytosine. In another embodiment of this disclosure there is provided an aqueous protein composition wherein the protein has a pH of 6 at the desired temperature, and the protein composition comprises at least one displacement buffer having a pKa that is 5 or less and preferably such displacement buffer is lactate.
In one embodiment of this disclosure there is provided an aqueous protein composition that is a hepatitis B vaccine preferably wherein the pH of the protein comprising the hepatitis B vaccine is 5 at the desired temperature and preferably wherein at least one displacement buffer has a pKa that is 6 or greater or at least one displacement buffer has a pKa of 4 or less or any combination of such displacement buffers are present in the composition, wherein the one or more displacement buffers are preferably selected from TRIS and histidine.
In one embodiment of this disclosure, the aqueous protein composition comprises glucose oxidase having a pH of 5 at the desired temperature and the protein composition further comprises at least one displacement buffer having a pKa that is 6 or greater wherein such displacement buffer includes but is not limited to TRIS. In another embodiment of this disclosure, the aqueous protein composition comprises glucose oxidase having a pH of 5 at the desired temperature and the protein composition further comprises at least one displacement buffer having a pKa that is 4 or less wherein such displacement buffer includes, but is not limited to, lactate.
In one embodiment of this disclosure, the aqueous protein composition comprises catalase having a pH of 6.7 at the desired temperature and the protein composition further comprises at least one displacement buffer having a pKa that is 7.7 or greater wherein such displacement buffer includes but is not limited to TRIS and lysine. In another embodiment of this disclosure, the aqueous protein composition comprises catalase having a pH of 6.7 at the desired temperature and the protein composition further comprises at least one displacement buffer having a pKa that is 5.7 or less wherein such displacement buffer includes, but is not limited to, lactate and lysine.
In another embodiment of this disclosure, the aqueous protein composition comprises uricase having a pH of 8.6 at the desired temperature and the protein composition further comprises at least one displacement buffer having a pKa that is 7.3 or less wherein such displacement buffer includes, but it not limited to succinate, glycine and cytosine.

In one embodiment of this disclosure there is provided an aqueous composition comprising a protein and one or more displacement buffers, wherein each displacement buffer has a pKa that is at least 1 unit greater or less than the pH of the composition and more preferably at least 2 units greater or less than the pH of the composition, with the proviso that said composition is substantially free of a conventional buffer having a pKa of the pH composition, wherein the composition preferably comprises less than about 1 mM of conventional buffer and wherein the composition may further comprise additional excipients suitable for stabilizing the protein and the composition including but not limited to stabilizing agents, protease inhibitors, chelating agents, preservatives, sugars and detergents.

In one embodiment of this disclosure there is provided an aqueous composition having a pH of about 5, comprising glucose oxidase and at least one additive selected from the group consisting of TRIS and lactate. In another embodiment of this disclosure there is providedprovides an aqueous composition having a pH of about 6.7, comprising catalase and at least one additive selected from the group consisting of TRIS, lysine and lactate. In yet another embodiment of this disclosure there is provided an aqueous composition having a pH of about 8.3, comprising uricase and at least one additive selected from the group consisting of succinate, lysine and lactate. In another embodiment of this disclosure there is provided an aqueous composition having a pH of about 5, comprising Hepatitis B antigen and at least one additive selected from the group consisting of TRIS, histidine and lactate. In yet another embodiment of this disclosure there is provided an aqueous composition having a pH of about 6, comprising human growth hormone and at least one additive selected from the group consisting of TRIS, cytosine, purine and lactate.

In another embodiment of this disclosure there is provided an aqueous composition comprising a protein and at least one additive, the composition being adjusted to a pH between 4 to 5, substantially free of a buffer having a pKa within one pH unit of said pH, wherein at least one additive is selected from the group consisting of Histidine, Maleate, Sulphite, Cyclamate, Hydrogen sulphate, Serine, Arginine, Lysine, Purine, Asparagine, Methionine, Threonine, Tyrosine, Isoleucine, Valine, Leucine, Alanine, Glycine, Tryptophan, Gentisate, Salicylate or salts thereof.

In another embodiment of this disclosure there is provided an aqueous composition comprising a protein and at least one additive, the composition being adjusted to pH between 4 to 5, substantially free of a buffer having a pKa within one pH unit of said pH, wherein at least one additive is selected from the group consisting of Maleate, Sulphite, 2-(N-Morpholino)ethanesulphonic acid, Bicarbonate, Histidine, Bis(2-hydroxyethyl) iminotris(hydroxymethyl)methane, N-(2-Acetamido)-2, iminodiacetic acid, 2-[(2-amino-2-oxoethyl)amino]ethanesulphonic acid, Piperazine, N,N'-bis(2-ethanesulphonic acid), Phosphate, N,N-Bis(2-hydroxyethyl)-2, aminoethanesulphonic acid, 3-[N,N-Bis(2-hydroxyethyl)amino]-2, hydroxypropanesulphonic acid, Triethanolamine, Piperazine-N,N'-bis(2, hydroxypropanesulphonic acid) or salts thereof.

In another embodiment of this disclosure there is provided an aqueous composition comprising a protein and at least one additive, the composition being adjusted to pH between 4.5 to 5.5, substantially free of a buffer having a pKa within one pH unit of said pH, wherein at least one additive is selected from the group consisting of Histidine, Maleate, Sulphite, Cyclamate, Hydrogen sulphate, Serine, Arginine, Lysine, Purine, Asparagine, Methionine, Threonine, Tyrosine, Isoleucine, Valine, Leucine, Alanine, Glycine, Tryptophan, Gentisate, Salicylate, Glyoxylate, Aspartame, Glucuronate or salts thereof.

In yet another embodiment of this disclosure there is provided an aqueous composition comprising a protein and at least one additive, the composition being adjusted to pH between 4.5 to 5.5, substantially free of a buffer having a pKa within one pH unit of said pH, wherein at least one additive is selected from the group consisting of Sulphite, Aspartame, Bis(2-hydroxyethyl) iminotris(hydroxymethyl)methane, N-(2-Acetamido)-2, iminodiacetic acid, 2-[(2-amino-2-oxoethyl)amino]ethanesulphonic acid, Piperazine, N,N'-bis(2-ethanesulphonic acid), Phosphate, N,N-Bis(2-hydroxyethyl)-2, aminoethanesulphonic acid, 3-[N,N-Bis(2-hydroxyethyl)amino]-2, hydroxypropanesulphonic acid, Triethanolamine, Piperazine-N,N'-bis(2, hydroxypropanesulphonic acid), Tris(hydroxymethyl)aminomethane, N, Tris(hydroxymethyl)glycine, N-Tris(hydroxymethyl)methyl-3-aminopropanesulphonic acid or salts thereof.

In one embodiment of this disclosure there is provided an aqueous composition, the composition being adjusted to a pH between 4.5 and 5.5, substantially free of a buffer having a pKa within one pH unit of said pH, comprising:

a. a protein selected from the group consisting of Interferon beta, Granulocyte-colony stimulating factor, Hepatitis B antigen, Hepatitis A and C vaccines or precursors or derivatives thereof,

b. at least one additive selected from the group consisting of Sulphite, Aspartame, Bis(2-hydroxyethyl) iminotris(hydroxymethyl)methane, N-(2-Acetamido)-2, iminodiacetic acid, 2-[(2-amino-2-oxoethyl)amino]ethanesulphonic acid, Piperazine, N,N'-bis(2-ethanesulphonic acid), Phosphate, N,N-Bis(2-hydroxyethyl)-2, aminoethanesulphonic acid, 3-[N,N-Bis(2-hydroxyethyl)amino]-2, hydroxypropanesulphonic acid, Triethanolamine, Piperazine-N,N'-bis(2, hydroxypropanesulphonic acid), Tris(hydroxymethyl)aminomethane, N, Tris(hydroxymethyl)glycine, N-Tris(hydroxymethyl)methyl-3-aminopropanesulphonic acid or salts thereof; and

c. at least one additive selected from the group consisting of Histidine, Maleate, Sulphite, Cyclamate, Hydrogen sulphate, Serine, Arginine, Lysine, Purine, Asparagine, Methionine, Threonine, Tyrosine, Isoleucine, Valine, Leucine, Alanine, Glycine, Tryptophan, Gentisate, Salicylate, Glyoxylate, Aspartame, Glucuronate or salts thereof

In yet another embodiment of this disclosure there is provided an aqueous composition comprising a protein and at least one additive, the composition being adjusted to pH between 5 to 6 substantially free of a buffer having a pKa within one pH unit of said pH, wherein at least one additive is selected from the group consisting of Aspartate, Serine, Arginine, Purine, Lysine, Asparagine, Methionine, Threonine, Tyrosine, Isoleucine, Valine, Leucine, Alanine, Glycine, Tryptophan, Gentisate, Salicylate, Glyoxylate, Aspartame, Glucuronate, Gluconate, Lactate, Glycolic acid or salts thereof.

In another embodiment of this disclosure there is provided an aqueous composition comprising a protein and at least one additive, the composition being adjusted to pH between 5 to 6, substantially free of a buffer having a pKa within one pH unit of said pH, wherein at least one additive is selected from the group consisting of Sulphite, Arginine, Purine, Asparagine, Threonine, Aspartame, Phosphate, N,N-Bis(2-hydroxyethyl)-2, aminoethanesulphonic acid, 3-[N,N-Bis(2-hydroxyethyl)amino]-2, hydroxypropanesulphonic acid, Triethanolamine, Piperazine-N,N'-bis(2, hydroxypropanesulphonic acid), Tris(hydroxymethyl)aminomethane, N, Tris(hydroxymethl)glycine, N-Tris(hydroxymethyl)methyl-3-aminopropanesulphonic acid or salts thereof.

In yet another embodiment of this disclosure there is provided an aqueous composition, the composition being adjusted to a pH between 5 and 6, substantially free of a buffer having a pKa within one pH unit of said pH, comprising:

 a. a protein selected from the group consisting of Hirudin, Iduronidase or precursors or derivatives thereof;
 b. at least one additive selected from the group consisting of Aspartate, Serine, Arginine, Purine, Lysine, Asparagine, Methionine, Threonine, Tyrosine, Isoleucine, Valine, Leucine, Alanine, Glycine, Tryptophan, Gentisate, Salicylate, Glyoxylate, Aspartame, Glucuronate, Gluconate, Lactate, Glycolic acid or salts thereof; and
 c. at least one additive selected from the group consisting of Sulphite, Arginine, Purine, Asparagine, Threonine, Aspartame, Phosphate, N,N-Bis(2-hydroxyethyl)-2, aminoethanesulphonic acid, 3-[N,N-Bis(2-hydroxyethyl)amino]-2, hydroxypropanesulphonic acid, Triethanolamine, Piperazine-N,N'-bis(2, hydroxypropanesulphonic acid), Tris(hydroxymethyl)aminomethane, N, Tris(hydroxymethl)glycine, N-Tris(hydroxymethyl)methyl-3-aminopropanesulphonic acid or salts thereof.

In yet another embodiment of this disclosure there is provided an aqueous composition comprising a protein and at least one additive, the composition being adjusted to pH between 5.5 to 6.5, substantially free of a buffer having a pKa within one pH unit of said pH, wherein at least one additive is selected from the group consisting of Aspartate, Glutamate, Gentisate, Tartrate, Salicylate, Glyoxylate, Aspartame, Glucuronate, Gluconate, Lactate, Glycolic acid, Adenine, Succinate, Ascorbate, Benzoate, Phenylacetate, Gallate, Cytosine or salts thereof.

In another embodiment of this disclosure there is provided an aqueous composition comprising a protein and at least one additive, the composition being adjusted to pH between 5.5 to 6.5, substantially free of a buffer having a pKa within one pH unit of said pH, wherein at least one additive is selected from the group consisting of Serine, Arginine, Lysine, Purine, Asparagine, Methionine, Threonine, Tyrosine, Tryptophan, Aspartame, 3-[N,N-Bis(2-hyroxyethyl)amino]-2, hydroxypropanesulphonic acid, Triethanolamine, Piperazine-N,N'-bis(2, hydroxypropanesulphonic acid), Tris(hydroxymethyl)aminomethane, N, Tris(hydroxymethyl)glycine, N-Tris(hydroxymethyl)methyl-3-aminopropanesulphonic, acid, Ammonium ion, Borate, 2-(N-Cyclohexylamino)ethanesulphonic acid, Triethanolamine or salts thereof

In another embodiment of this disclosure there is provided an aqueous composition, the composition being adjusted to a pH between 5.5 and 6.5, substantially free of a buffer having a pKa within one pH unit of said pH, comprising:

 a. a protein selected from the group consisting of Galactosidase, Glucocerebrosidase, Aprotinin, Collagenase, Human growth hormone, DNase I, Interleukin-1 receptor antagonist, Interferon alpha, monoclonal antibodies such as Anti-EGFR IgG, TNF binding IgG, Anti-CD20 antibody, Anti-VEGF antibody, Anti-RSV antibody Acellular pertussis vaccine, Dyptheria vaccine, HPV vaccine, TB vaccine or precursors or derivatives thereof;
 b. at least one additive selected from the group consisting of Aspartate, Glutamate, Gentisate, Tartrate, Salicylate, Glyoxylate, Aspartame, Glucuronate, Gluconate, Lactate, Glycolic acid, Adenine, Succinate, Ascorbate, Benzoate, Phenylacetate, Gallate, Cytosine or salts thereof; and
 c. at least one additive selected from the group consisting of Serine, Arginine, Lysine, Purine, Asparagine, Methionine, Threonine, Tyrosine, Tryptophan, Aspartame, 3-[N,N-Bis(2-hyroxyethyl)amino]-2, hydroxypropanesulphonic acid, Triethanolamine, Piperazine-N,N'-bis(2, hydroxypropanesulphonic acid), Tris(hydroxymethyl)aminomethane, N, Tris(hydroxymethyl)glycine, N-Tris(hydroxymethyl)methyl-3-aminopropanesulphonic, acid, Ammonium ion, Borate, 2-(N-Cyclohexylamino)ethanesulphonic acid, Triethanolamine or salts thereof.

In yet another embodiment of this disclosure there is provided an aqueous composition comprising a protein and at least one additive, the composition being adjusted to pH between 6 to 7, substantially free of a buffer having a pKa within one pH unit of said pH, wherein at least one additive is selected from the group consisting of Aspartate, Glutamate, Tartrate, Salicylate, Fumarate, Glyoxylate, Glucuronate, Gluconate, Lactate, Glycolic acid, Adenine, Succinate, Ascorbate, Benzoate, Phenylacetate, Gallate, Cytosine, p-Aminobenzoic acid, Sorbate, Acetate, Propionate, Alginate or salts thereof.

In another embodiment of this disclosure there is provided an aqueous composition comprising a protein and at least one additive, the composition being adjusted to pH between 6 to 7 substantially free of a buffer having a pKa within one pH unit of said pH, wherein at least one additive is selected from the group consisting of Aspartate, Serine, Arginine, Lysine, Purine, Asparagine, Glutamate, Methionine, Threonine, Tyrosine, Isoleucine, Valine, Leucine, Alanine, Glycine, Tryptophan, Adenine, p-Aminobenzoic acid, Tris(hydroxymethyl)aminomethane, N, Tris(hydroxymethyl)glycine, N-Tris(hydroxymethyl)methyl-3-aminopropanesulphonic, acid, Ammonium ion, Borate, 2-(N-Cyclohexylamino)ethanesulphonic acid, Triethanolamine, 2-Amino-2-methyl-1-propanol, Palmitate or salts thereof.

In yet another embodiment of this disclosure there is provided an aqueous composition, the composition being adjusted to a pH between 6 and 7, substantially free of a buffer having a pKa within one pH unit of said pH, comprising:

a. a protein selected from the group consisting of TNF receptor, Darbepoetin alpha, Alpha-1-antitrypsin inhibitor, Natriuretic peptide, protein C, Follicle-stimulating hormone, insulin, Insulin-like growth factor, Bone morphogenic proteins, Keratinocyte growth factor, Interleukin-2, Intergeron gamma, Rabies vaccine, Rotavirus vaccine, Tetanus toxoid or precursors or derivatives thereof;

b. at least one additive selected from the group consisting of Aspartate, Glutamate, Tartrate, Salicylate, Fumarate, Glyoxylate, Glucuronate, Gluconate, Lactate, Glycolic acid, Adenine, Succinate, Ascorbate, Benzoate, Phenylacetate, Gallate, Cytosine, p-Aminobenzoic acid, Sorbate, Acetate, Propionate, Alginate or salts thereof; and

c. wherein at least one additive selected from the group consisting of Aspartate, Serine, Arginine, Lysine, Purine, Asparagine, Glutamate, Methionine, Threonine, Tyrosine, Isoleucine, Valine, Leucine, Alanine, Glycine, Tryptophan, Adenine, p-Aminobenzoic acid, Tris(hydroxymethyl)aminomethane, N, Tris(hydroxymethyl)glycine, N-Tris(hydroxymethyl)methyl-3-aminopropanesulphonic, acid, Ammonium ion, Borate, 2-(N-Cyclohexylamino)ethanesulphonic acid, Triethanolamine, 2-Amino-2-methyl-1-propanol, Palmitate or salts thereof.

In yet another embodiment of this disclosure there is provided an aqueous composition comprising a protein and at least one additive, the composition being adjusted to pH between 6.5 to 7.5, substantially free of a buffer having a pKa within one pH unit of said pH, wherein at least one additive is selected from the group consisting of Aspartate, Glutamate, Tartrate, Fumarate, Malate, Gluconate, Lactate, Glycolic acid, Adenine, Succinate, Ascorbate, Benzoate, Phenylacetate, Glutarate, Gallate, Cytosine, p-Aminobenzoic acid, Sorbate, Acetate, Propionate, Alginate, Urate or salts thereof.

In another embodiment of this disclosure there is provided an aqueous composition comprising a protein and at least one additive, the composition being adjusted to pH between 6.5 to 7.5, substantially free of a buffer having a pKa within one pH unit of said pH, wherein at least one additive is selected from the group consisting of Aspartate, Serine, Arginine, Lysine, Purine, Asparagine, Glutamate, Methionine, Threonine, Tyrosine, Isoleucine, Valine, Leucine, Alanine, Glycine, Tryptophan, Adenine, p-Aminobenzoic acid, Ammonium ion, Borate, 2-(N-Cyclohexylamino)ethanesulphonic acid, Triethanolamine, 2-Amino-2-methyl-1-propanol, Palmitate, Creatinine or salts thereof.

In yet another embodiment of this disclosure there is provided an aqueous composition, the composition being adjusted to a pH between 6.5 and 7.5, substantially free of a buffer having a pKa within one pH unit of said pH, comprising:

a. a protein selected from the group consisting of Alfacept, Alteplase, Botulinum toxin, Parathyroid hormone, Human chorionic gonadotropin, Thyroid stimulating hormone, Calcitonin, Erythropoietin, Haemophilus b vaccine, Japanese Encephalitis vaccine, Staphylococcus vaccine, malaria vaccine or precursors or derivatives thereof;

b. wherein at least one additive selected from the group consisting of Aspartate, Glutamate, Tartrate, Fumarate, Malate, Gluconate, Lactate, Glycolic acid, Adenine, Succinate, Ascorbate, Benzoate, Phenylacetate, Glutarate, Gallate, Cytosine, p-Aminobenzoic acid, Sorbate, Acetate, Propionate, Alginate, Urate or salts thereof; and

c. wherein at least one additive selected from the group consisting of Aspartate, Serine, Arginine, Lysine, Purine, Asparagine, Glutamate, Methionine, Threonine, Tyrosine, Isoleucine, Valine, Leucine, Alanine, Glycine, Tryptophan, Adenine, p-Aminobenzoic acid, Ammonium ion, Borate, 2-(N-Cyclohexylamino)ethanesulphonic acid, Triethanolamine, 2-Amino-2-methyl-1-propanol, Palmitate, Creatinine or salts thereof.

In an aspect of the invention, the sealed container further comprises an aqueous system comprising a protein, characterised in that

a. the system does not comprise a meaningful amount of conventional buffer, i.e. compound with $pK_a$ close to the pH of the composition at the intended temperature range of storage of the composition; and

b. the pH of the composition is set to a value at which the composition has maximum measurable stability with respect to pH.

In one embodiment, a system of the sealed container according to the invention preferably comprises a pH within a range of $\pm 0.5$ pH units and preferably $\pm 1$ pH units of the pH at which the composition has maximum measurable stability with respect to pH.

In another embodiment of the invention, the system of the sealed container of the invention does not comprise any compound with $pK_a$ within 1 unit from pH of the composition at the intended temperature range of storage of the composition at concentration higher than 500 $\mu$M.

In accordance with the invention, the system of the sealed container of the invention may further comprise: a polyalcohol preferably at about at least 0.5% (w/w); an inorganic salt; a preservative; a protease inhibitor a surfactant; a chelating agent or any combination thereof.

In one embodiment, the system of the sealed container of the invention comprises a protein that is preferably in its native state. In another embodiment, the system of the sealed container of the invention comprises a protein selected from the group consisting of a hormone or growth factor, an enzyme, an antibody, an interferon, an immunogenic protein, or any combination thereof. In yet another embodiment, the system of the sealed container of the invention is preferably an aqueous solution, suspension or dispersion. In one embodiment the system of the sealed container of the invention comprises a solid adsorbent. In one preferred embodiment the system of the sealed container of the invention comprises a solid absorbent wherein the solid adsorbent includes but is not limited to a vaccine adjuvant such as alumina.

In accordance with the invention, a system or composition of the sealed container of the invention is suitable for use in therapy or diagnosis practised on the human or animal body.

The invention is further illustrated by the following non-limiting examples.

Examples

The following Examples illustrate the invention.

Materials

Boric acid (Fluka, Code 15660)
Catalase (from bovine liver, Sigma C9322, 2380 U /mg solid)
Citric acid (Fisher, Code C/6200/53)
Cytosine (Sigma, Code C3506)
Deionised water (conductivity < 10 $\mu$S cm$^{-1}$; either analytical reagent grade, Fisher or Sanyo Fistreem MultiPure)
Disodium hydrogen orthophosphate (Fisher, Code S/4520/53)
Di-sodium maleate (Sigma, Code M9009)
Di-sodium malate (Aldrich, Code 233935)
DMSO - Dimethyl sulfoxide (Sigma-Aldrich Code154938-500)
Glucose (Fisher, Code G050061)
Glucose Oxidase (Biocatalysts G575P ~150 U /mg solid)
Hepatitis B recombinant vaccine (Shantha)
Human growth hormone (Somatropin) standard was supplied by National Institute of
Biological Standards and Control (Potters Bar, UK). Further samples for experimentation were obtained on prescription from a local GP surgery.
Hydrochloric acid (Fisher, Code J/4310/17)
Hydrogen peroxide (Sigma, Code H1009)
D,L-Lactic acid (Fluka, Code 1077141)
Lactoperoxidase (from bovine milk, DMV International: 1,050 units mg$^{-1}$ by ABTS method pH 5.0)
Lysine (Sigma, Code L5501)
Nicotinic acid (Sigma, Code N4126)
PBS - Phosphate buffered saline (Sigma D1408)
Potassium iodide (Fisher, Code 5880/53)
Sodium chloride (Fisher, Code C/3160/63)
Sodium citrate (Sigma, Code S1804)
Sodium hydroxide (Fisher, Code J/7800/15)
Sodium dihydrogen orthophosphate (Fisher, Code S/3760/60)

Sodium lactate (Fluka, Code 71723)
Sodium urate (Sigma, Code U2875)
Starch (Acros Organics, Code 177132500)
Succinic acid (Fluka, Code 14079)
TMB - Tetramethylbenzidine (Sigma T-2885)
TRIS base - Tris(hydroxymethyl)aminomethane (Fisher Bioreagents, CodeBPE152-1)
Uricase (Sigma, Code U0880)

Unless stated otherwise, phosphate buffers of given concentration and pH (X mM, pH $\underline{Y}$) used in this work were prepared by mixing disodium hydrogen orthophosphate ($\underline{X}$ mM) with sodium dihydrogen orthophosphate (X mM) to achieve the required pH $\underline{Y}$.

Unless stated otherwise, citrate/phosphate buffers of given concentration and pH ($\underline{X}$ mM, pH $\underline{Y}$) used in this work were prepared by mixing di-sodium hydrogen orthophosphate ($\underline{X}$ mM) with citric acid ($\underline{X}$ mM) to achieve the required pH Y.

Overall Experimental Plan

In each example, an aqueous solution of a given enzyme was prepared with selected additives in a 2 mL glass vial. Each solution was assayed for protein activity or structural integrity. The vials were then sealed and incubated at a given temperature for a given period of time. The solution was then assayed again and the recovery of activity or structural integrity was expressed as the percentage of the original (i.e. preincubation) result. Temperatures above ambient were used so as to be more demanding than work at ambient, and to provide more quickly an indication of protein stability.

Example 1 - Glucose oxidase (from Penicillium sp.) at 59 °C

Stability of glucose oxidase was tested in aqueous solutions at 350 $\mu$g mL$^{-1}$ concentration. Stability was compared between solutions prepared both in the presence and in the absence of conventional buffers, and in the presence of displaced buffers. In each case the pH of the formulation was optimal with respect to stability of glucose oxidase in that particular formulation. The stability was compared in the following formulations:

- 10 mM citrate (pH 5.4); prepared by mixing sodium citrate (10 mM) with citric acid (10 mM) to achieve the required pH; glucose oxidase was added to this formulation to achieve 350 $\mu$g mL$^{-1}$ concentration, pH was checked after glucose oxidase addition and, if necessary, adjusted to 5.4 with either hydrochloric acid (5 M) or sodium hydroxide (5 M).
- 10 mM nicotinate (pH 5.2); prepared by dissolving nicotinic acid (10 mM) in water and adjusting pH with sodium hydroxide (5 M); glucose oxidase was added to this formulation to achieve 350 $\mu$g mL$^{-1}$ concentration, pH was checked after glucose oxidase addition and, if necessary, adjusted to 5.2 with either hydrochloric acid (5 M) or sodium hydroxide (5 M).
- 10 mM lactate (pH 5.0); prepared by mixing sodium lactate (10 mM) with lactic acid (10 mM) to achieve the required pH; glucose oxidase was added to this formulation to achieve 350 $\mu$g mL$^{-1}$ concentration, pH was checked after glucose oxidase addition and, if necessary, adjusted to 5.0 with either hydrochloric acid (5 M) or sodium hydroxide (5 M).
- 10 mM TRIS (pH 5.3); prepared by dissolving Trizma base (10 mM) in water and adjusting pH with hydrochloric acid (5 M); glucose oxidase was added to this formulation to achieve 350 $\mu$g mL$^{-1}$ concentration, pH was checked after glucose oxidase addition and, if necessary, adjusted to 5.3 with either hydrochloric acid (5 M) or sodium hydroxide (5 M).
- 10 mM lactate + 10 mM TRIS (pH 5.0); prepared by dissolving lactic acid (10 mM) and Trizma base (10 mM) in water and adjusting pH with either hydrochloric acid (5 M) or sodium hydroxide (5 M); glucose oxidase was added to this formulation to achieve 350 $\mu$g mL$^{-1}$ concentration, pH was checked after glucose oxidase addition and, if necessary, adjusted to 5.0 with either hydrochloric acid (5 M) or sodium hydroxide (5 M).
- Neat protein (pH 4.9); prepared by dissolving glucose oxidase (350 $\mu$g mL$^{-1}$) directly in water and adjusting pH with hydrochloric acid (10 mM).

The solutions were incubated at 59 °C for 22 hours and then assayed for remaining glucose oxidase activity. This was performed according to the following procedure: 50 $\mu$L of the sample (containing 350 $\mu$g mL$^{-1}$ of glucose oxidase) was added to 50 mL of deionised water. The following reagents were then added: 10 mL of reagent mix (5.5 parts of 0.1 M sodium dihydrogen orthophosphate, pH 6 + 4 parts 2% w/w starch + 0.5 part of 1 mg/mL lactoperoxidase enzyme); 5 mL of 100 mM potassium iodide and 5 mL of 20% w/w glucose solution. These were mixed together quickly. Time = 0 was counted from the addition of the glucose. After 5 min, 1 ml of 5 M hydrochloric acid was added to stop the reaction. The absorbance was then read at 630 nm using a Unicam UV-visible spectrophotometer. If the colour intensity was too great to allow an accurate reading, the sample was diluted with a defined volume of deionised water to bring the colour back on scale. The results were expressed as percentage recovery, by reference to the absorbance measured in the fresh samples (i.e. prior to incubation at increased temperature).

All formulations were tested at their optimum pH with respect to glucose oxidase stability. Nicotinate ($pK_a$=4.90) and citrate ($pK_a1$ = 3.14, $pK_a2$ = 4.78, $pK_a3$ = 6.39) were tested as the conventional buffers showing recovery of glucose oxidase activity 25% (citrate) and 23% (nicotinate) after incubation at 59 °C for 22 hours. Considerably higher recovery was observed in the absence of conventional buffer. The recovery was about 54% if the protein was incubated in pure water adjusted to pH 4.9. Even better recovery was observed in the presence of one component with $pK_a$ at least one unit higher than the pH of the composition. In the presence of TRIS ($pK_a$=8.30) 68% recovery was achieved and in the presence of purine ($pK_a$ = 8.90) 72% recovery was achieved. Similarly, better stability was achieved in the presence of one component with $pK_a$ at least one unit lower than the pH of the composition - in the presence of lactate ($pK_a$ =3.85) 69% recovery was achieved. 73% recovery was achieved in the two component displaced buffer mixture containing lactate ($pK_a$ = 3.85) and TRIS ($pK_a$ = 8.30). The two-component displaced buffer formulation was therefore optimal, because it resulted in the best stability of the enzyme and it ensured better pH stability due to a buffering effect both on the acidic and on the alkaline side of the pH of the composition.

Example 2 - Glucose oxidase (from Penicillium sp.) at 40 °C

Stability of glucose oxidase was tested in aqueous solutions at 350 $\mu$g mL$^{-1}$ concentration. Stability was compared between solutions prepared both in the presence and in the absence of conventional buffers, and in the presence of displaced buffers. In each case the pH of the formulation was optimal with respect to stability of glucose oxidase in that particular formulation. The stability was compared in the following formulations:

- 10 mM citrate (pH 5.2); prepared by mixing sodium citrate (10 mM) with citric acid (10 mM) to achieve the required pH; glucose oxidase was added to this formulation to achieve 350 $\mu$g mL$^{-1}$ concentration, pH was checked after glucose oxidase addition and, if necessary, adjusted to 5.2 with either hydrochloric acid (5 M) or sodium hydroxide (5 M).
- 200 mM citrate (pH 5.0); prepared by mixing sodium citrate (200 mM) with citric acid (200 mM) to achieve the required pH; glucose oxidase was added to this formulation to achieve 350 $\mu$g mL$^{-1}$ concentration, pH was checked after glucose oxidase addition and, if necessary, adjusted to 5.0 with either hydrochloric acid (5 M) or sodium hydroxide (5 M).
- 10 mM succinate (pH 5.2); prepared by dissolving succinic acid (10 mM) in water and adjusting pH with sodium hydroxide (5 M); glucose oxidase was added to this formulation to achieve 350 $\mu$g mL$^{-1}$ concentration, pH was checked after glucose oxidase addition and, if necessary, adjusted to 5.2 with either hydrochloric acid (5 M) or sodium hydroxide (5 M).
- 200 mM succinate (pH 5.2); prepared by dissolving succinic acid (200 mM) in water and adjusting pH with sodium hydroxide (5 M); glucose oxidase was added to this formulation to achieve 350 $\mu$g mL$^{-1}$ concentration, pH was checked after glucose oxidase addition and, if necessary, adjusted to 5.2 with either hydrochloric acid (5 M) or sodium hydroxide (5 M).
- 10 mM nicotinate (pH 5.2); prepared by dissolving nicotinic acid (10 mM) in water and adjusting pH with sodium hydroxide (5 M); glucose oxidase was added to this formulation to achieve 350 $\mu$g mL$^{-1}$ concentration, pH was checked after glucose oxidase addition and, if necessary, adjusted to 5.2 with either hydrochloric acid (5 M) or sodium hydroxide (5 M).
- 10 mM lactate (pH 4.7); prepared by mixing sodium lactate (10 mM) with lactic acid (10 mM) to achieve the required pH; glucose oxidase was added to this formulation to achieve 350 $\mu$g mL$^{-1}$ concentration, pH was checked after glucose oxidase addition and, if necessary, adjusted to 4.7 with either hydrochloric acid (5 M) or sodium hydroxide (5 M).
- 200 mM lactate (pH 4.7); prepared by mixing sodium lactate (200 mM) with lactic acid (200 mM) to achieve the required pH; glucose oxidase was added to this formulation to achieve 350 $\mu$g mL$^{-1}$ concentration, pH was checked after glucose oxidase addition and, if necessary, adjusted to 4.7 with either hydrochloric acid (5 M) or sodium hydroxide (5 M).
- 10 mM TRIS (pH 5.0); prepared by dissolving Trizma base (10 mM) in water and adjusting pH with hydrochloric acid (5 M); glucose oxidase was added to this formulation to achieve 350 $\mu$g mL$^{-1}$ concentration, pH was checked after glucose oxidase addition and, if necessary, adjusted to 5.0 with either hydrochloric acid (5 M) or sodium hydroxide (5 M).

- 200 mM TRIS (pH 5.5); prepared by dissolving Trizma base (200 mM) in water and adjusting pH with hydrochloric acid (5 M); glucose oxidase was added to this formulation to achieve 350 $\mu$g mL$^{-1}$ concentration, pH was checked after glucose oxidase addition and, if necessary, adjusted to 5.5 with either hydrochloric acid (5 M) or sodium hydroxide (5 M).
- 10 mM lactate + 10 mM TRIS (pH 4.7); prepared by dissolving lactic acid (10 mM) and Trizma base (10 mM) in water and adjusting pH with either hydrochloric acid (5 M) or sodium hydroxide (5 M); glucose oxidase was added to this formulation to achieve 350 $\mu$g mL$^{-1}$ concentration, pH was checked after glucose oxidase addition and, if necessary, adjusted to 4.7 with either hydrochloric acid (5 M) or sodium hydroxide (5 M).
- 200 mM lactate + 200 mM TRIS (pH 5.3); prepared by dissolving lactic acid (200 mM) and Trizma base (200 mM) in water and adjusting pH with either hydrochloric acid (5 M) or sodium hydroxide (5 M); glucose oxidase was added to this formulation to achieve 350 $\mu$g mL$^{-1}$ concentration, pH was checked after glucose oxidase addition and, if necessary, adjusted to 5.3 with either hydrochloric acid (5 M) or sodium hydroxide (5 M).

The solutions were incubated at 40 °C for 26 weeks and then assayed for remaining glucose oxidase activity. This was performed according to the procedure described in Example 1.

All formulations were tested at their optimum pH with respect to glucose oxidase stability. Nicotinate ($pK_a$=4.90), citrate ($pK_a$1 = 3.14, $pK_a$2 = 4.78, $pK_a$3 = 6.39) and succinate ($pK_a$1 = 4.16, $pK_a$2 = 5.61) were tested as the conventional buffers showing, at 10 mM concentration, recovery of glucose oxidase activity 0% (10 mM citrate), 52% (10 mM nicotinate) and 47% (10 mM succinate) after incubation at 40 °C for 26 weeks. Considerably better recovery of glucose oxidase activity was observed in the absence of conventional buffers and in the presence of at least one displaced buffer. The recovery was 91% in the presence of 10 mM lactate ($pK_a$ = 3.85) and 70% in the presence of 10 mM TRIS ($pK_a$ = 8.30). The presence of both TRIS (10 mM) and lactate (10 mM) resulted in 90% recovery. At 200 mM concentrations of conventional buffers the recovery was as follows: citrate - 46%, succinate - 55%. At 200 mM of displaced buffers the recovery was as follows: lactate - 76%, TRIS - 91 %, TRIS & lactate - 92%. The two-component displaced buffer formulation was therefore optimal, because it resulted in best stability of the enzyme at both concentrations tested and it ensured better pH stability due to a buffering effect both on the acidic and on the alkaline side of the pH of the composition.

Example 3 - Catalase (from bovine liver) at 52 °C

Stability of catalase was tested in aqueous solutions at 100 $\mu$g mL$^{-1}$ concentration. Stability was compared between solutions prepared both in the presence and in the absence of conventional buffers, and in the presence of displaced buffers. In each case the pH of the formulation was optimal with respect to stability of catalase in that particular formulation. The stability was compared in the following formulations:

- 10 mM citrate (pH 6.4); prepared by mixing sodium citrate (10 mM) with citric acid (10 mM) to achieve the required pH; catalase was added to this formulation to achieve 100 $\mu$g mL$^{-1}$ concentration, pH was checked after catalase addition and, if necessary, adjusted to 6.4 with either hydrochloric acid (5 M) or sodium hydroxide (5 M).
- 10 mM maleate (pH 6.5); prepared by dissolving sodium maleate (10 mM) in water and adjusting pH with hydrochloric acid (5 M); catalase was added to this formulation to achieve 100 $\mu$g mL$^{-1}$ concentration, pH was checked after catalase addition and, if necessary, adjusted to 6.5 with either hydrochloric acid (5 M) or sodium hydroxide (5 M).
- 10 mM lactate (pH 6.4); prepared by mixing sodium lactate (10 mM) with lactic acid (10 mM) to achieve the required pH; catalase was added to this formulation to achieve 100 $\mu$g mL$^{-1}$ concentration, pH was checked after catalase addition and, if necessary, adjusted to 6.4 with either hydrochloric acid (5 M) or sodium hydroxide (5 M).
- 10 mM TRIS (pH 6.7); prepared by dissolving Trizma base (10 mM) in water and adjusting pH with hydrochloric acid (5 M); catalase was added to this formulation to achieve 100 $\mu$g mL$^{-1}$ concentration, pH was checked after catalase addition and, if necessary, adjusted to 6.7 with either hydrochloric acid (5 M) or sodium hydroxide (5 M).
- 10 mM lactate + 10 mM TRIS (pH 6.9); prepared by dissolving lactic acid (10 mM) and Trizma base (10 mM) in water and adjusting pH with either hydrochloric acid (5 M) or sodium hydroxide (5 M); catalase was added to this formulation to achieve 100 $\mu$g mL$^{-1}$ concentration, pH was checked after catalase addition and, if necessary, adjusted to 6.9 with either hydrochloric acid (5 M) or sodium hydroxide (5 M).
- Neat protein (pH 6.8); prepared by dissolving catalase (100 $\mu$g mL$^{-1}$) directly in water and adjusting pH with hydrochloric acid (10 mM).

The solutions were incubated at 52 °C for 42 hours and then assayed for remaining catalase activity. This was performed according to the following procedure: 2 mL of hydrogen peroxide (30 mM in water) was added to 18 mL of PBS in a 125 mL polypropylene pot. 100 $\mu$L of sample (containing 100 $\mu$g mL$^{-1}$ catalase) was added and mixed. The resulting mixture was incubated at room temperature precisely for 30 min. In the meantime, the following reagents were mixed in a plastic cuvette for spectrophotometric measurements:

- 2.73 mL of citrate/phosphate buffer (0.1 M, pH 5.0)
- 100 $\mu$L of TMB (3 mg /mL, dissolved in DMSO)
- 100 $\mu$L of lactoperoxidase

Following the 30 min incubation period, 70 $\mu$L of the catalase containing mixture was added to the cuvette and absorbance was read in approximately 30 s. The results were expressed as percentage recovery, by reference to the absorbance measured in the fresh samples (i.e. prior to incubation at increased temperature).

Citrate ($pK_a1 = 3.14$, $pK_a2 = 4.78$, $pK_a3 = 6.39$) and maleate ($pK_a1 = 1.83$, $pK_a2 = 6.20$) were tested as the conventional buffers showing recovery of catalase activity of 12% (citrate) and 13% (maleate) after incubation at 52 °C for 42 hours. Considerably improved stability was observed in the absence of conventional buffers. The recovery was 48% if the protein was incubated in pure water with pH adjusted to pH 6.8. Similar recovery was observed in the presence of components with $pK_a$ at least one unit higher or one unit lower than the pH of the composition. The recovery was as follows: 45% in the presence of TRIS ($pK_a = 8.30$), 44% in the presence of lactate ($pK_a = 3.85$), 39% in the presence of purine ($pK_a = 8.9$), 47% in the presence of both TRIS ($pK_a = 8.30$) and lactate ($pK_a = 3.85$). Although the recovery was comparable between the excipient-free formulation (i.e. water only with pH adjustment) and formulations containing displaced buffers it is still preferable to use the displaced buffers because of better control over pH in their presence.

Example 4 - Catalase (from bovine liver) at 40 °C

Stability of catalase was tested in aqueous solutions at 100 $\mu$g mL$^{-1}$ concentration. Stability was compared between solutions prepared both in the presence and in the absence of conventional buffers, and in the presence of displaced buffers. In each case the pH of the formulation was optimal with respect to stability of catalase in that particular formulation. The stability was compared in the following formulations (all formulations contained 200 mM NaCl to improve solubility of the enzyme):

- 10 mM citrate (pH 6.8); prepared by mixing sodium citrate (10 mM) with citric acid (10 mM) to achieve the required pH; catalase was added to this formulation to achieve 100 $\mu$g mL$^{-1}$ concentration, and sodium chloride was added to achieve 200 mM concentration; pH was checked after catalase and sodium chloride addition and, if necessary, adjusted to 6.8 with either hydrochloric acid (5 M) or sodium hydroxide (5 M).
- 10 mM maleate (pH 6.8); prepared by dissolving sodium maleate (10 mM) in water and adjusting pH with hydrochloric acid (5 M); catalase was added to this formulation to achieve 100 $\mu$g mL$^{-1}$ concentration, and sodium chloride was added to achieve 200 mM concentration; pH was checked after catalase and sodium chloride addition and, if necessary, adjusted to 6.8 with either hydrochloric acid (5 M) or sodium hydroxide (5 M).
- 10 mM phosphate (pH 6.8); prepared by dissolving sodium dihydrogen phosphate (10 mM) in water and adjusting pH with sodium hydroxide (5 M); catalase was added to this formulation to achieve 100 $\mu$g mL$^{-1}$ concentration, and sodium chloride was added to achieve 200 mM concentration; pH was checked after catalase and sodium chloride addition and, if necessary, adjusted to 6.8 with either hydrochloric acid (5 M) or sodium hydroxide (5 M).
- 10 mM lactate (pH 6.4); prepared by mixing sodium lactate (10 mM) with lactic acid (10 mM) to achieve the required pH; catalase was added to this formulation to achieve 100 $\mu$g mL$^{-1}$ concentration, and sodium chloride was added to achieve 200 mM concentration; pH was checked after catalase and sodium chloride addition and, if necessary, adjusted to 6.4 with either hydrochloric acid (5 M) or sodium hydroxide (5 M).
- 10 mM purine (pH 6.4); prepared by dissolving purine (10 mM) in water and adjusting pH with hydrochloric acid (5 M); catalase was added to this formulation to achieve 100 $\mu$g mL$^{-1}$ concentration, and sodium chloride was added to achieve 200 mM concentration; pH was checked after catalase and sodium chloride addition and, if necessary, adjusted to 6.4 with either hydrochloric acid (5 M) or sodium hydroxide (5 M).
- 10 mM lysine (pH 6.6): prepared by dissolving lysine (10 mM) in water and adjusting pH with hydrochloric acid (5 M); catalase was added to this formulation to achieve 100 $\mu$g mL$^{-1}$ concentration, and sodium chloride was added to achieve 200 mM concentration; pH was checked after catalase and sodium chloride addition and, if necessary, adjusted to 6.6 with either hydrochloric acid (5 M) or sodium hydroxide (5 M).
- 10 mM lactate + 10 mM purine (pH 7.0); prepared by dissolving lactic acid (10 mM) and purine (10 mM) in water and adjusting pH with either hydrochloric acid (5 M) or sodium hydroxide (5 M); catalase was added to this formulation to achieve 100 $\mu$g mL$^{-1}$ concentration, and sodium chloride was added to achieve 200 mM concentration; pH was checked after catalase and sodium chloride addition and, if necessary, adjusted to 7.0 with either hydrochloric acid (5 M) or sodium hydroxide (5 M).
- Neat protein (pH 6.4); prepared by dissolving catalase (100 $\mu$g mL$^{-1}$) directly in 200 mM sodium chloride and adjusting pH with hydrochloric acid (10 mM)

The solutions were incubated at 40 °C for 7 days and then assayed for remaining catalase activity. This was performed according to the procedure described in Example 3.

Citrate ($pK_a1 = 3.14$, $pK_a2 = 4.78$, $pK_a3 = 6.39$), maleate ($pK_a1 = 1.83$, $pK_a2 = 6.20$) and phosphate ($pK_a1 = 2.16$, $pK_a2 = 7.10$) were tested as the conventional buffers showing recovery of catalase activity of 4% (citrate or maleate) and 2% (phosphate) after incubation at 40 °C for 7 days. Recovery of catalase activity was equally poor (3%) in the absence of conventional buffer (i.e. in sodium chloride solution adjusted to pH 6.4). Considerably improved stability was observed in displaced buffer formulations. The recovery was 11% in the presence of lysine ($pK_a1 = 2.25$ $pK_a2 = 9.2$, $pK_a3 = 10.8$) 58% in the presence of lactate ($pK_a = 3.85$), 63% in the presence of purine ($pK_a = 8.90$) and 61 % in the presence of both lactate and purine. The displaced buffer formulations are thus the best choice to ensure stability of catalase.

<u>Example 5 - Uricase</u>

Stability of uricase was tested in aqueous solutions at 250 $\mu$g mL$^{-1}$. Stability was compared between solutions prepared both in the presence and in the absence of conventional buffers, and in the presence of displaced buffers. In each case the pH of the formulation was optimal with respect to stability of uricase in that particular formulation. The stability was compared in the following formulations:

- 20 mM borate (pH 8.6); prepared by dissolving boric (20 mM) in water and adjusting pH with sodium hydroxide (5 M); uricase was added to this formulation to achieve 250 $\mu$g mL$^{-1}$ concentration, pH was checked after uricase addition and, if necessary, adjusted to 8.6 with either hydrochloric acid (5 M) or sodium hydroxide (5 M).
- 20 mM purine (pH 8.6); prepared by dissolving purine (20 mM) in water and adjusting pH with sodium hydroxide (5 M); uricase was added to this formulation to achieve 250 $\mu$g mL$^{-1}$ concentration, pH was checked after uricase addition and, if necessary, adjusted to 8.6 with either hydrochloric acid (5 M) or sodium hydroxide (5 M).
- 20 mM succinate (pH 8.6); prepared by dissolving succinic acid (20 mM) in water and adjusting pH with sodium hydroxide (5 M); uricase was added to this formulation to achieve 250 $\mu$g mL$^{-1}$ concentration, pH was checked after uricase addition and, if necessary, adjusted to 8.6 with either hydrochloric acid (5 M) or sodium hydroxide (5 M).
- 20 mM cytosine (pH 9.0); prepared by dissolving cytosine (20 mM) in water and adjusting pH with sodium hydroxide (5 M); uricase was added to this formulation to achieve 250 $\mu$g mL$^{-1}$ concentration, pH was checked after uricase addition and, if necessary, adjusted to 9.0 with either hydrochloric acid (5 M) or sodium hydroxide (5 M).
- 20 mM glycine (pH 9.0); prepared by dissolving glycine (20 mM) in water and adjusting pH with either hydrochloric acid (5 M) or sodium hydroxide (5 M); uricase was added to this formulation to achieve 250 $\mu$g mL$^{-1}$ concentration, pH was checked after uricase addition and, if necessary, adjusted to 9.0 with either hydrochloric acid (5 M) or sodium hydroxide (5 M).

The solutions were incubated at 60 °C for 18 hours and then assayed for remaining uricase activity. This was performed according to the following procedure: The following solutions were mixed in a 1 cm cuvette:

- 1.5 mL of borate buffer (25 mM, pH 8.5); prepared by adjusting the pH of 25 mM boric acid using sodium hydroxide (5 M)
- 0.8 mL of sodium urate (2 mM)

40 $\mu$L of sample (containing 250 $\mu$g mL$^{-1}$ of uricase) was added and mixed quickly. Time = 0 was counted from the addition of the uricase. After 5 min, the following reagents were added in this particular order (the first reagent should be added at exactly 5 min, the timing of the other reagents addition is less crucial):

- 0.8 mL of citrate/phosphate buffer (0.5 M, pH 4.0); prepared by mixing 0.5 M citric acid with 0.5 M disodium hydrogen phosphate to achieve the pH required
- 100 $\mu$L of TMB (3 mg mL$^{-1}$, dissolved in DMSO)
- 100 $\mu$L of lactoperoxidase (1 mg mL$^{-1}$, dissolved in water)

The resulting solution was mixed thoroughly and absorbance was read at 630 nm using a Unicam UV-visible spectrophotometer (type: Helios gamma). The results were expressed as percentage recovery, by reference to the absorbance measured in the fresh samples (i.e. prior to incubation at increased temperature).

Borate ($pK_a = 9.27$), and purine ($pK_a = 8.90$) were tested as the conventional buffers showing maximum recovery of uricase 48% (borate) and 52% (purine) after incubation at 60 °C for 18 hours. Better stability of uricase was observed in the absence of conventional buffers and presence of displaced buffers. The recovery was 72% in the presence of succinate ($pK_a1 = 4.16$, $pK_a2 = 5.51$), 64% in the presence of glycine ($pK_a1 = 2.43$ $pK_a2 = 9.84$), and 78% in the presence of cytosine ($pK_a1 = 4.5$, $pK_a2 = 12.2$). The use of displaced buffers is thus optimal to ensure stability of uricase.

Example 6 - Hepatitis B recombinant vaccine

Stability of Hepatitis B recombinant vaccine was tested in the presence of appropriate adjuvant (aluminium hydroxide suspension) in aqueous solutions at 20 $\mu$g mL$^{-1}$ of protein and 0.5 mg mL$^{-1}$ of aluminium hydroxide. These concentrations are the same as those in a commercial vaccine product. Stability was compared between solutions prepared in the presence of conventional buffers, and in the presence of displaced buffers. In each case the pH of the formulation was optimal with respect to stability of Hepatitis B antigen in that particular formulation. The stability was compared in the following formulations (All formulations contained 40 mM sodium phosphate to ensure optimal binding of the vaccine onto the alumina):

- 40 mM succinate (pH 5.0); prepared by dissolving succinic acid (40 mM) and sodium dihydrogen phosphate (40 mM) in water and adjusting pH with either hydrochloric acid (5 M) or sodium hydroxide (5 M); hepatitis B antigen adsorbed on aluminium hydroxide adjuvant was added to the formulation to achieve 20 $\mu$g mL$^{-1}$ of protein and 0.5 mg mL$^{-1}$ of aluminium hydroxide in the formulation.

- 10 mM malate (pH 5.0); prepared by dissolving sodium malate (40 mM) and sodium dihydrogen phosphate (40 mM) in water and adjusting pH with either hydrochloric acid (5 M) or sodium hydroxide (5 M); hepatitis B antigen adsorbed on aluminium hydroxide adjuvant was added to the formulation to achieve 20 $\mu$g mL$^{-1}$ of protein and 0.5 mg mL$^{-1}$ of aluminium hydroxide in the formulation.

- 40 mM lactate (pH 5.0); prepared by dissolving sodium lactate (40 mM) and sodium dihydrogen phosphate (40 mM) in water and adjusting pH with either hydrochloric acid (5 M) or sodium hydroxide (5 M); hepatitis B antigen adsorbed on aluminium hydroxide adjuvant was added to the formulation to achieve 20 $\mu$g mL$^{-1}$ of protein and 0.5 mg mL$^{-1}$ of aluminium hydroxide in the formulation.

- 40 mM lactate + 40 mM TRIS (pH 5.0); prepared by dissolving lactic acid (40 mM), Trizma base (40 mM) and sodium dihydrogen phosphate (40 mM) in water and adjusting pH with either hydrochloric acid (5 M) or sodium hydroxide (5 M); hepatitis B antigen adsorbed on aluminium hydroxide adjuvant was added to the formulation to achieve 20 $\mu$g mL$^{-1}$ of protein and 0.5 mg mL$^{-1}$ of aluminium hydroxide in the formulation.

- 40 mM histidine (pH 5.0); prepared by dissolving histidine (40 mM) and sodium dihydrogen phosphate (40 mM) in water and adjusting pH with either hydrochloric acid (5 M) or sodium hydroxide (5 M); hepatitis B antigen adsorbed on aluminium hydroxide adjuvant was added to the formulation to achieve 20 $\mu$g mL$^{-1}$ of protein and 0.5 mg mL$^{-1}$ of aluminium hydroxide in the formulation.

The solutions were incubated at 55 °C for 4 weeks and then assayed for remaining antigenic activity. The antigenic activity of the Hepatitis B vaccine was measured using the AUSZYME monoclonal diagnostic kit (Abbott Laboratories; cat no. 1980-64). The antigenic activity was determined both in the whole vaccine and in the supernatant following centrifugation (13,000 RPM, 5 min). The antigenic activity was expressed as a percentage with respect to the value measured of the untreated refrigerated vaccine.

Succinate ($pK_a1$ = 4.16, $pK_a2$ = 5.51) and malate ($pK_a1$ = 3.40, $pK_a2$ = 5.11) were tested as the conventional buffers showing maximum recovery of Hepatitis B antigen 64% (succinate) and 57% (malate) after incubation at 55 °C for 4 weeks. Considerably better stability was observed in the presence of displaced buffers: 88% in the presence of TRIS ($pK_a$ = 8.10), 91% in the presence of lactate ($pK_a$ = 3.85), 93% in the combined presence of TRIS and lactate and 98% in the presence of histidine ($pK_a1$ = 1.78, $pK_a2$ = 6.10, $pK_a3$ = 9.26).

Example 7 -Human growth hormone

Stability of human growth hormone was tested in aqueous solutions at 1.25 mg mL$^{-1}$. Stability was compared between solutions prepared both in the presence of conventional buffers and in the presence of displaced buffers. In each case the pH of the formulation was optimal with respect to stability of human growth hormone in that particular formulation. The stability was compared in the following formulations:

- 20 mM citrate (pH 6.0); prepared by mixing citric acid (20 mM) with sodium citrate to achieve the required pH; human growth hormone was added to this formulation to achieve 1.25 mg mL$^{-1}$ concentration.

- 20 mM TRIS (pH 6.0); prepared by dissolving Trizma base (20 mM) in water and adjusting pH with hydrochloric acid (5 M); human growth hormone was added to this formulation to achieve 1.25 mg mL$^{-1}$ concentration.

- 20 mM lactate (pH 6.0); prepared by mixing sodium lactate (10 mM) with lactic acid (10 mM) to achieve the required pH; human growth hormone was added to this formulation to achieve 1.25 mg mL$^{-1}$ concentration.

- 20 mM lactate + 20 mM TRIS (pH 6.0); prepared by dissolving lactic acid (20 mM) and Trizma base (20 mM) in water and adjusting pH with either hydrochloric acid (5 M) or sodium hydroxide (5 M); human growth hormone was added to this formulation to achieve 1.25 mg mL$^{-1}$ concentration.
- 20 mM cytosine (pH 6.0); prepared by dissolving cytosine (20 mM) in water and adjusting pH with sodium hydroxide (5 M); human growth hormone was added to this formulation to achieve 1.25 mg mL$^{-1}$ concentration.
- 20 mM purine (pH 6.0); prepared by dissolving purine (20 mM) in water and adjusting pH with either hydrochloric acid (5 M) or sodium hydroxide (5 M); human growth hormone was added to this formulation to achieve 1.25 mg mL$^{-1}$ concentration.
- 10 mM cytosine + 10 mM Purine (pH 6.0); prepared by co-dissolving cytosine (10 mM) and purine (10 mM) and adjusting pH with sodium hydroxide (5 M); human growth hormone was added to this formulation to achieve 1.25 mg mL$^{-1}$ concentration.

The solutions were incubated at 40 °C for 5 weeks and then assayed for remaining intact protein using the following reversed-phase HPLC method: Mobile phase was prepared by mixing 71 parts (by volume) of a solution of TRIS (0.05 M, in water adjusted with hydrochloric acid to a pH of 7.5) and 29 parts (by volume) of n-propylalcohol. The mobile phase was filtered prior to its use. The liquid chromatograph (Agilent 1100 series) was equipped with a 214 nm detector and a 4.6 × 250 mm column (Phenomenex 00G-4167-E0) packed with butylsilyl silica gel with a granulometry of 5 $\mu$m and a porosity of 30 nm, maintained at 45°C. The flow rate was maintained at 0.5 mL min$^{-1}$. 15 $\mu$L of aqueous samples of human growth hormone (typically 1-2.5 mg mL$^{-1}$) were injected.

Citrate (pK$_a$1 = 3.14, pK$_a$2 = 4.78, pK$_a$3 = 6.39) was tested as the conventional buffer showing structural recovery of human growth hormone 5% after incubation at 40 °C for 5 weeks. Considerably better recovery of human growth hormone was observed in the presence of displaced buffers: 39.8% in the presence of TRIS (pK$_a$ = 8.10), 38.2% in the presence of lactate (pK$_a$ = 3.85), 42.2% in the presence of TRIS & lactate, 51.3% in the presence of cytosine (pK$_a$1 = 4.5, pK$_a$2 = 12.2), 49.1% in the presence of purine (pK$_a$ = 8.90) and 48.1 % in the presence of purine & cytosine.

**Claims**

1. A sealed container containing an aqueous composition comprising a protein and two displacement buffers, wherein one displacement buffer has a pKa that is at least 1 unit greater than the pH of the composition and one displacement buffer has a pKa that is at least 1 unit less than the pH of the composition, said pH of the composition being set to a value at which the composition has maximum measurable stability with respect to pH, wherein one of the displacement buffers is TRIS, with the proviso that said composition contains not more than 2 mM of a conventional buffer having a pKa that is within one pH unit of the pH of the composition, and wherein each displacement buffer is present at a concentration of 2 to 200 mM.

2. A sealed container of claim 1 wherein the aqueous composition comprises a surfactant.

3. A sealed container of claim 1 or claim 2 wherein each displacement buffer of the aqueous composition has a pKa that is 1 to 5 units greater or less than the pH of the composition.

4. A sealed container of claim 3 wherein each displacement buffer of the aqueous composition has a pKa that is 1 to 4 units greater or less than the pH of the composition.

5. A sealed container of any one of claims 1 to 4, wherein each displacement buffer of the aqueous composition is present at a concentration of 5 to 100 mM.

6. A sealed container of any one of claims 1 to 5, wherein in the aqueous composition

     (i) the protein is Hepatitis B antigen and the pH is about 5; or
     (ii) the protein is human growth hormone and the pH is about 6.

7. A sealed container of any one of claims 1 to 5, wherein in the aqueous composition the protein is a therapeutic enzyme.

8. A sealed container of any one of claims 1 to 5, wherein in the aqueous composition the protein is a therapeutic antibody.

9. A sealed container of any one of claims 1 to 5, wherein in the aqueous composition the protein is human growth hormone.

**10.** A sealed container of any one of claims 1 to 5, wherein in the aqueous composition the protein is a vaccine.

**11.** A sealed container of any one of claims 1 to 5, wherein in the aqueous composition the protein is a blood coagulation factor.

**12.** A sealed container of claim 11, wherein in the aqueous composition the protein is a blood coagulation factor selected from Factor VIII and Factor IX.

**13.** A sealed container of any one of claims 1 to 5, wherein in the aqueous composition the protein is insulin.

**14.** A sealed container of any one of claims 1 to 5, wherein in the aqueous composition the protein is interferon.

**15.** A sealed container of any of claims 1 to 14, wherein the aqueous composition is for use in therapy or diagnosis practised on the human or animal body.


**Patentansprüche**

**1.** Verschlossenes Behältnis, das eine wässrige Zusammensetzung, die ein Protein und zwei Verdrängungspuffer umfasst, enthält, wobei ein Verdrängungspuffer einen pKa-Wert aufweist, der um mindestens eine Einheit höher als der pH-Wert der Zusammensetzung ist und ein Verdrängungspuffer einen pKa-Wert aufweist, der um mindestens eine Einheit niedriger als der pH-Wert der Zusammensetzung ist, wobei der pH-Wert der Zusammensetzung auf einen Wert eingestellt ist, bei dem die Zusammensetzung eine maximale messbare pH-Stabilität aufweist, wobei es sich bei einem der Verdrängungspuffer um TRIS handelt, mit der Maßgabe, dass die Zusammensetzung nicht mehr als 2 mM eines traditionellen Puffers mit einem pKa-Wert, der innerhalb einer pH-Einheit des pH-Werts der Zusammensetzung liegt, aufweist, und wobei jeder Verdrängungspuffer in einer Konzentration von 2 bis 200 mM vorliegt.

**2.** Verschlossenes Behältnis nach Anspruch 1, wobei die wässrige Zusammensetzung ein Tensid umfasst.

**3.** Verschlossenes Behältnis nach Anspruch 1 oder Anspruch 2, wobei jeder Verdrängungspuffer der wässrigen Zusammensetzung einen pKa-Wert aufweist, der um 1 bis 5 Einheiten höher oder niedriger als der pH-Wert der Zusammensetzung ist.

**4.** Verschlossenes Behältnis nach Anspruch 3, wobei jeder Verdrängungspuffer der wässrigen Zusammensetzung einen pKa-Wert aufweist, der um 1 bis 4 Einheiten höher oder niedriger als der pH-Wert der Zusammensetzung ist.

**5.** Verschlossenes Behältnis nach einem der Ansprüche 1 bis 4, wobei jeder Verdrängungspuffer der wässrigen Zusammensetzung in einer Konzentration von 5 bis 100 mM vorliegt.

**6.** Verschlossenes Behältnis nach einem der Ansprüche 1 bis 5, wobei in der wässrigen Zusammensetzung

(i) das Protein Hepatitis-B-Antigen ist und der pH-Wert ungefähr 5 beträgt; oder
(ii) das Protein menschliches Wachstumshormon ist und der pH-Wert ungefähr 6 beträgt.

**7.** Verschlossenes Behältnis nach einem der Ansprüche 1 bis 5, wobei in der wässrigen Zusammensetzung das Protein ein therapeutisches Enzym ist.

**8.** Verschlossenes Behältnis nach einem der Ansprüche 1 bis 5, wobei in der wässrigen Zusammensetzung das Protein ein therapeutischer Antikörper ist.

**9.** Verschlossenes Behältnis nach einem der Ansprüche 1 bis 5, wobei in der wässrigen Zusammensetzung das Protein ein menschliches Wachstumshormon ist.

**10.** Verschlossenes Behältnis nach einem der Ansprüche 1 bis 5, wobei in der wässrigen Zusammensetzung das Protein ein Impfstoff ist.

**11.** Verschlossenes Behältnis nach einem der Ansprüche 1 bis 5, wobei in der wässrigen Zusammensetzung das Protein ein Blutgerinnungsfaktor ist.

**12.** Verschlossenes Behältnis nach Anspruch 11, wobei in der wässrigen Zusammensetzung das Protein ein Blutgerinnungsfaktor, ausgewählt aus Faktor VIII und Faktor IX, ist.

**13.** Verschlossenes Behältnis nach einem der Ansprüche 1 bis 5, wobei in der wässrigen Zusammensetzung das Protein Insulin ist.

**14.** Verschlossenes Behältnis nach einem der Ansprüche 1 bis 5, wobei in der wässrigen Zusammensetzung das Protein Interferon ist.

**15.** Verschlossenes Behältnis nach einem der Ansprüche 1 bis 14, wobei die wässrige Zusammensetzung zur Verwendung in der Therapie oder Diagnose, die am menschlichen oder tierischen Körper durchgeführt wird, dient.

**Revendications**

**1.** Récipient scellé contenant une composition aqueuse comprenant une protéine et deux tampons de déplacement, un tampon de déplacement ayant un pKa qui est supérieur d'au moins 1 unité au pH de la composition et un tampon de déplacement ayant un pKa qui est inférieur d'au moins 1 unité au pH de la composition, ledit pH de la composition étant défini à une valeur à laquelle la composition a une stabilité mesurable maximale vis-à-vis du pH, un des tampons de déplacement étant TRIS, à condition que ladite composition ne contienne pas plus de 2 mM d'un tampon conventionnel ayant un pKa qui diffère de pas plus d'une unité de pH du pH de la composition, et chaque tampon de déplacement étant présent à une concentration de 2 à 200 mM.

**2.** Récipient scellé de la revendication 1 dans lequel la composition aqueuse comprend un tensioactif.

**3.** Récipient scellé de la revendication 1 ou la revendication 2 dans lequel chaque tampon de déplacement de la composition aqueuse a un pKa qui est supérieur ou inférieur de 1 à 5 unités au pH de la composition.

**4.** Récipient scellé de la revendication 3 dans lequel chaque tampon de déplacement de la composition aqueuse a un pKa qui est supérieur ou inférieur de 1 à 4 unités au pH de la composition.

**5.** Récipient scellé de l'une quelconque des revendications 1 à 4, dans lequel chaque tampon de déplacement de la composition aqueuse est présent à une concentration de 5 à 100 mM.

**6.** Récipient scellé de l'une quelconque des revendications 1 à 5, dans lequel, dans la composition aqueuse,

(i) la protéine est un antigène de l'hépatite B et le pH est d'environ 5 ; ou
(ii) la protéine est l'hormone de croissance humaine et le pH est d'environ 6.

**7.** Récipient scellé de l'une quelconque des revendications 1 à 5, dans lequel, dans la composition aqueuse, la protéine est une enzyme thérapeutique.

**8.** Récipient scellé de l'une quelconque des revendications 1 à 5, dans lequel, dans la composition aqueuse, la protéine est un anticorps thérapeutique.

**9.** Récipient scellé de l'une quelconque des revendications 1 à 5, dans lequel, dans la composition aqueuse, la protéine est l'hormone de croissance humaine.

**10.** Récipient scellé de l'une quelconque des revendications 1 à 5, dans lequel, dans la composition aqueuse, la protéine est un vaccin.

**11.** Récipient scellé de l'une quelconque des revendications 1 à 5, dans lequel, dans la composition aqueuse, la protéine est un facteur de coagulation sanguine.

**12.** Récipient scellé de la revendication 11, dans lequel, dans la composition aqueuse, la protéine est un facteur de coagulation sanguine choisi parmi le facteur VIII et le facteur IX.

**13.** Récipient scellé de l'une quelconque des revendications 1 à 5, dans lequel, dans la composition aqueuse, la protéine est l'insuline.

**14.** Récipient scellé de l'une quelconque des revendications 1 à 5, dans lequel, dans la composition aqueuse, la protéine est l'interféron.

**15.** Récipient scellé de l'une quelconque des revendications 1 à 14, dans lequel la composition aqueuse est pour utilisation en thérapie ou en diagnostic pratiqué sur le corps humain ou animal.

**FIGURE 1**

**FIGURE 2**

**FIGURE 3**

**EP 2 114 456 B1**

## REFERENCES CITED IN THE DESCRIPTION

**Patent documents cited in the description**

- EP 1314437 A
- GB 2006002470 W
- EP 884053 A
- US 4476118 A